# EUROPÄISCHE PATENTSCHRIFT

(11) **EP 1 999 103 B1**
(45) Veröffentlichungstag und Bekanntmachung des Hinweises auf die Patenterteilung: **29.07.2009**
(21) Anmeldenummer: 07711952.7
(22) Anmeldetag: 15.03.2007
(51) Int. Cl.: C07C 217/00

(54) **ETHANOLAMIN-GLYCERINETHER**
ETHANOLAMINE GLYCERYL ETHERS
ETHER DE GLYCERINE-ETHANOLAMINE

(30) Priorität: 22.03.2006 DE 102006013058
(43) Veröffentlichungstag der Anmeldung: 10.12.2008
(73) Patentinhaber: Clariant Finance (BVI) Limited, Road Town, Tortola (VG)
(72) Erfinder: SCHERL, Franz-Xaver, 84508 Burgkirchen (DE); HESS, Joachim, 65719 Hofheim (DE); ZERRER, Ralf, 79400 Kandern (DE); SOWA, Christian, 67435 Neustadt/Weinstrasse (DE)
(74) Vertreter: Paczkowski, Marcus
(86) Internationale Anmeldenummer: PCT/EP2007/002273
(87) Internationale Veröffentlichungsnummer: WO 2007/107282

(56) Entgegenhaltungen:
- US-A- 2 505 374

## Beschreibung

Gegenstand der Erfindung sind Ethanolamin-Glycerinether Kondensationsprodukte und deren Verwendung in Dispersionen.

Dispersionen sind Stoffsysteme, bestehend aus zwei oder mehreren Phasen, bei dem ein Stoff (die dispergierte oder disperse Phase) in einem anderen (dem Dispergiermittel oder Dispergens) in feinster Form verteilt, d.h. dispergiert, ist. Sowohl das Dispergiermittel, als auch die Stoffe der dispersen Phase können fest, flüssig oder gasförmig sein. Nach dem Grad der Verteilung unterscheidet man nach molekulardispersen Systemen, kolloiddispersen Systemen, feindispersen Systemen und grobdispersen Systemen. Beispiele für Dispersionen sind Suspensionen, Emulsionen, Schäume, Aerosole und Rauch. Viele technische Produkte werden in Form von Dispersionen angewendet.

Um stabile Dispersionen, insbesondere Suspensionen oder Emulsionen zu erhalten, müssen häufig grenzflächenaktive Stoffe (Dispergatoren oder Emulgatoren) zugesetzt werden, die die Verteilung der Phasen erst ermöglichen oder erleichtern und einer Gelbildung, Phasentrennung, Kristallisation oder Sedimentation der einzelnen Komponenten entgegenwirken.

Es bestand die Aufgabe, neue effiziente Dispergatoren bzw. Emulgatoren, die technisch leicht zugänglich, für unterschiedliche Stoffsysteme einsetzbar, aber auch toxikologisch unbedenklich und ökologisch verträglich sind, zur Verfügung zu stellen.

Überraschend wurde gefunden, dass diese Aufgabe durch spezielle Ethanolamin-Glycerinether Kondensationsprodukte gelöst wird. Diese zeigen insbesondere hervorragende grenzflächenaktive Eigenschaften, sind gut einsetzbar in wässrigen Dispersionen, Suspensionskonzentraten und Suspoemulsionen, aber auch in Emulsionen und Emulsionskonzentraten, bewirken eine hohe Kompatibilität der Komponenten, beispielsweise Adjuvants, Dispergiermittel, Elektrolyte etc. untereinander und eine hohe Suspensibilität (Schwebefähigkeit) der Komponenten, sowie eine Stabilisierung der Phasen.

Gegenstand der Erfindung sind Ethanolamin-Glycerinether Kondensationsprodukte der Formel (I) 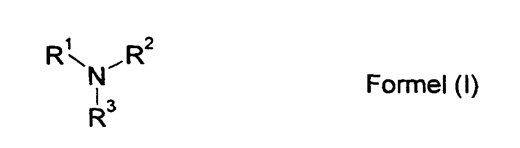 worin
R¹, R² und R³ jeweils unabhängig voneinander gleich oder verschieden sein können und
für Wasserstoff,
für eine lineare oder verzweigte, gesättigte Alkylkette mit 1 bis 20, vorzugsweise 3 bis 20 und besonders bevorzugt 4 bis 18 Kohlenstoffatomen,
für eine lineare oder verzweigte ungesättigte Alkenylgruppe mit 2 bis 20, vorzugsweise 3 bis 20 und besonders bevorzugt 4 bis 18 Kohlenstoffatomen, für -CH₂-CH₂-OH oder
für einen Rest der Formel (II) 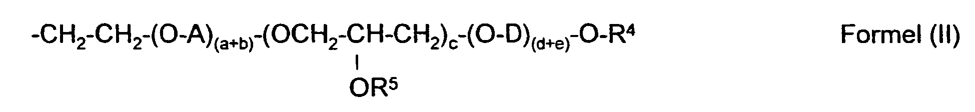 stehen,
worin
- (O-A): für eine Alkylenoxygruppe ausgewählt aus Ethylenoxygruppe (EO), Propylenoxygruppe (PO), Butylenoxygruppe und/oder Phenyloxirangruppe,
- a: für eine Zahl von 0 bis 50, vorzugsweise 0 bis 20, besonders bevorzugt 0 bis 10,
- b: für eine Zahl von 0 bis 50, vorzugsweise 0 bis 20, besonders bevorzugt 0 bis 10,
- R⁵: für Wasserstoff und/oder
für (O-A)ₐH, worin (O-A) für eine Alkylenoxygruppe ausgewählt aus Ethylenoxygruppe (EO), Propylenoxygruppe (PO), Butylenoxygruppe und/oder Phenyloxirangruppe und a für eine Zahl von 1 bis 30, stehen und/oder
für -(CR⁶R⁷)ₐ-Phenyl, wobei R⁶ und R⁷, die gleich oder verschieden sein können, für -H, für eine OH-Gruppe, für lineares oder verzweigtes (C₁-C₁₀)-Alkyl oder für lineares oder verzweigtes (C₂-C₃₀)-Alkenyl stehen und a für eine Zahl von 1 bis 10 steht, und/oder
für -(CR⁶R⁷)ₐ-Naphtyl, wobei R⁶ und R⁷, die gleich oder verschieden sein können, für -H, für eine OH-Gruppe, für lineares oder verzweigtes (C₁-C₁₀)-Alkyl oder für lineares oder verzweigtes (C₂-C₃₀)-Alkenyl stehen und a für eine Zahl von 1 bis 10 steht, und/oder
für eine Gruppe der Formel (III) 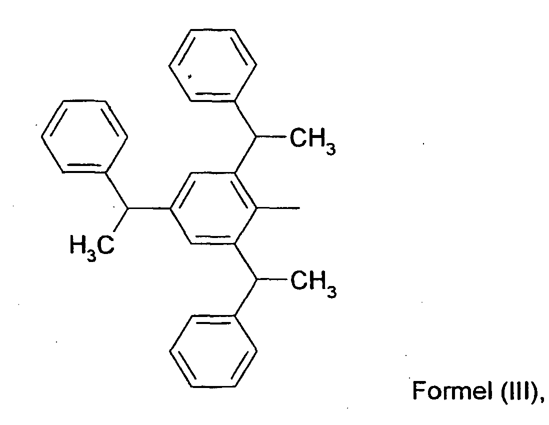
und/oder
für R⁸R⁹N-(CH₂)_{b}-, wobei R⁸ und R⁹, die gleich oder verschieden sein können, für -H, lineares oder verzweigtes (C₁-C₁₀)-Alkyl oder lineares oder verzweigtes (C₂-C₃₀)-Alkenyl stehen und b für eine Zahl von 1 bis 22 steht, und/oder
für HO-(CH₂)_{b}-, wobei b für eine Zahl von 1 bis 22 steht, und/oder
für -SO₃⁻X⁺, -PO₃²⁻X⁺X'⁺ oder -CH₂COO⁻X⁺, wobei X⁺ und X'⁺ für H⁺, Li⁺, Na⁺, K⁺, Ca²⁺/2, Mg²⁺/2 oder N(R⁸)₄⁺ stehen und die R⁸ gleich oder verschieden sein können und für -H oder (C₁-C₁₀)-Alkyl, vorzugsweise (C₁-C₄)-Alkyl, stehen, und/oder
für eine Gruppe der Formel (IV) 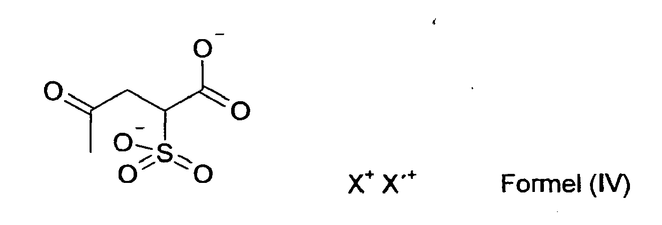 wobei X⁺ und X'⁺ für H⁺, Li⁺, Na⁺, K⁺, Ca²⁺/2, Mg²⁺/2 oder N(R⁸)₄⁺ stehen und die R⁸ gleich oder verschieden sein können und für -H oder (C₁-C₁₀)-Alkyl, vorzugsweise (C₁-C₄)-Alkyl, stehen, und/oder
für -C(R¹¹)₂-COO⁻X⁺, -CO-R¹²-COO⁻X⁺ oder -C(R¹¹)₂C(R¹¹)₂C(R¹¹)₂-N(R¹³)₂, wobei die R¹¹ gleich oder verschieden sein können und für -H und/oder -CH₃ stehen, R¹² für (C₁-C₁₀)-Alkylen oder (C₂-C₃₀)-Alkenylen steht, die R¹³ gleich oder verschieden sein können und für (C₁-C₁₀)-Alkyl oder (C₂-C₃₀)-Alkenyl stehen, X⁺ für H⁺, Li⁺, Na⁺, K⁺, Ca²⁺/2, Mg²⁺/2 oder N(R⁸)₄⁺ steht und die R⁸ gleich oder verschieden sein können und für -H oder (C₁-C₁₀)-Alkyl, vorzugsweise für (C₁-C₄)-Alkyl, stehen, und/oder
für -C(R¹⁴)₂C(R¹⁴)₂C(R¹⁴)₂-N((GO)₂H)₂, wobei die R¹⁴ gleich oder verschieden sein können und für -H und/oder -CH₃ stehen, G für -C₂H₄-, -C₃H₆- oder -C₄H₈- und z für eine Zahl von 1 bis 22 steht, und/oder
für eine Gruppe der Formel (V) 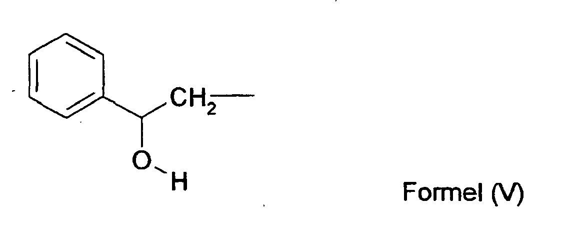 steht,
- c: für eine Zahl von 1 bis 25, vorzugsweise für eine Zahl von 1 bis 20,
- (O-D): für eine Alkylenoxygruppe ausgewählt aus Ethylenoxygruppe (EO), Propylenoxygruppe (PO), Butylenoxygruppe und/oder Phenyloxirangruppe,
- d: für eine Zahl von 0 bis 50, vorzugsweise 0 bis 20, besonders bevorzugt 0 bis 10,
- e: für eine Zahl von 0 bis 50, vorzugsweise 0 bis 20, besonders bevorzugt 0 bis 10, stehen und
- R⁴: die Bedeutung von R⁵ haben kann,
mit der Maßgabe, dass mindestens einer der Reste R¹, R² und R³ ein Rest der Formel (II) ist und mindestens ein weiterer der Reste R¹, R² und R³ ausgewählt ist aus -CH₂-CH₂-OH und Resten der Formel (II).

Im Rahmen der vorliegenden Erfindung können die Alkenylgruppen ein- oder mehrfach ungesättigt sein.

In einer bevorzugten Ausführungsform der Erfindung sind mindestens zwei der Reste R¹, R² und R³ Reste der Formel (II).

In einer weiteren bevorzugten Ausführungsform der Erfindung sind zwei der Reste R¹, R² und R³ ausgewählt aus -CH₂-CH₂-OH und Resten der Formel (II). In diesen Verbindungen ist demnach einer der Reste R¹, R² und R³ ein Rest der Formel (II) und ein weiterer der Reste R¹, R² und R³ ausgewählt aus -CH₂-CH₂-OH und Resten der Formel (II). Unter diesen Verbindungen sind wiederum diejenigen bevorzugt, worin zwei der Reste R¹, R² und R³ Reste der Formel (II) sind. Der dritte der Reste R¹, R² und R³ steht für Wasserstoff oder für eine lineare oder verzweigte, gesättigte Alkylkette mit 1 bis 20, vorzugsweise 3 bis 20 und besonders bevorzugt 4 bis 18 Kohlenstoffatomen oder für eine lineare oder verzweigte ungesättigte Alkenylgruppe mit 2 bis 20, vorzugsweise 3 bis 20 und besonders bevorzugt 4 bis 18 Kohlenstoffatomen, vorzugsweise für eine lineare oder verzweigte, gesättigte Alkylkette mit 1 bis 20, vorzugsweise 3 bis 20 und besonders bevorzugt 4 bis 18 Kohlenstoffatomen oder für eine lineare oder verzweigte ungesättigte Alkenylgruppe mit 2 bis 20, vorzugsweise 3 bis 20 und besonders bevorzugt 4 bis 18 Kohlenstoffatomen, und besonders bevorzugt für eine lineare oder verzweigte, gesättigte Alkylkette mit 1 bis 20, vorzugsweise 3 bis 20 und besonders bevorzugt 4 bis 18 Kohlenstoffatomen.

In einer weiteren bevorzugten Ausführungsform der Erfindung sind alle drei Reste R¹, R² und R³ ausgewählt aus -CH₂-CH₂-OH und Resten der Formel (II). In diesen Verbindungen ist demnach mindestens einer der Reste R¹, R² und R³ ein Rest der Formel (II) und die beiden anderen der Reste R¹, R² und R³ ausgewählt aus -CH₂-CH₂-OH und Resten der Formel (II). In einer insbesondere bevorzugten Ausführungsform der Erfindung ist einer der Reste R¹, R² und R³ ein Rest der Formel (II) und die beiden anderen der Reste R¹, R² und R³ sind Reste der Formel -CH₂-CH₂-OH. In einer weiteren insbesondere bevorzugten Ausführungsform der Erfindung sind zwei der Reste R¹, R² und R³ Reste der Formel (II) und der dritte der Reste R¹, R² und R³ ein Rest der Formel -CH₂-CH₂-OH. In einer weiteren insbesondere bevorzugten Ausführungsform der Erfindung sind alle drei Reste R¹, R² und R³ Reste der Formel (II).

Ein weiterer bevorzugter Gegenstand der Erfindung sind Ethanolamin-Glycerinether Kondensationsprodukte der Formel (I), **dadurch gekennzeichnet, dass** (O-A) und (O-D) jeweils für eine einheitliche Alkylenoxygruppe, (a+b) für eine Zahl von 0 bis 20 und (d+e) für eine Zahl von 0 bis 20 stehen.

Ein weiterer bevorzugter Gegenstand der Erfindung sind Ethanolamin-Glycerinether Kondensationsprodukte der Formel (I), **dadurch gekennzeichnet, dass** (O-A)_{(a+b)} und (O-D)_{(d+e)} für gemischte Alkylenoxygruppen der Formel -(O-A¹)ₐ-(O-A²)_{b}- bzw. -(O-D¹)_{d}-(O-D²)ₑ-, die statistisch oder blockweise angeordnet sein können, stehen und in denen a und b jeweils eine Zahl von 1 bis 30 bedeuten sowie d und e jeweils eine Zahl von 1 bis 20 bedeuten, (O-A¹) und (O-D¹) für eine Ethylenoxyeinheit, und (O-A²) und (O-D²) für eine Propylenoxyeinheit, für eine Butylenoxyeinheit, für eine Phenyloxiraneinheit, oder für Mischungen aus diesen stehen.

Ein weiterer bevorzugter Gegenstand der Erfindung sind Ethanolamin-Glycerinether Kondensationsprodukte der Formel (I), **dadurch gekennzeichnet,** dass die Reste R¹, R² und R³ gleich sind und für eine Gruppe der Formel (IIa) stehen, 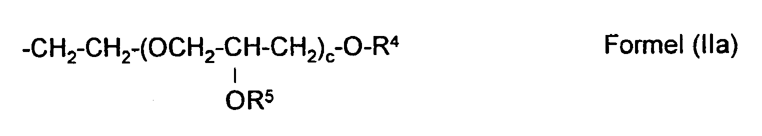 worin
- R⁵: Wasserstoff ist,
- c: für eine Zahl von 1 bis 25, bevorzugt 1 bis 20, besonders bevorzugt 2 bis 15, insbesondere bevorzugt 4 bis 12, außerordentlich bevorzugt 6 bis 10 steht, und
- R⁴: Wasserstoff ist.

Ein weiterer bevorzugter Gegenstand der Erfindung sind Ethanolamin-Glycerinether Kondensationsprodukte der Formel (I), **dadurch gekennzeichnet, dass** die Reste R¹, R² und R³ gleich sind und für eine Gruppe der Formel (IIa) stehen, 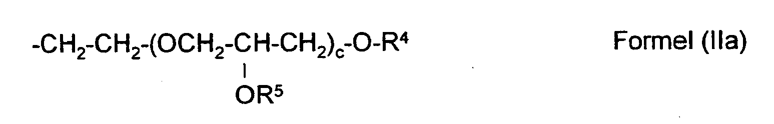 worin
- R⁵: Wasserstoff ist,
- c: für eine Zahl von 1 bis 25, bevorzugt 1 bis 20, besonders bevorzugt 2 bis 15, insbesondere bevorzugt 4 bis 12, außerordentlich bevorzugt 6 bis 10 steht, und
- R⁴: für eine Gruppe der Formel (III) 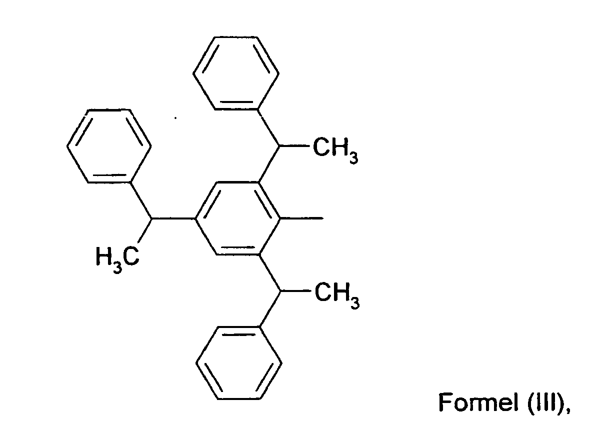 steht.

Ein weiterer bevorzugter Gegenstand der Erfindung sind Ethanolamin-Glycerinether Kondensationsprodukte der Formel (I), **dadurch gekennzeichnet, dass** die Reste R¹, R² und R³ gleich sind und für eine Gruppe der Formel (IIb) stehen 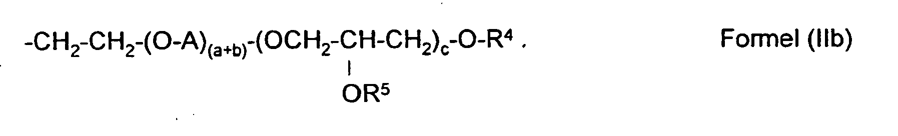 worin
(O-A)_{(a+b)} für eine einheitliche Alkylenoxygruppe steht und (a+b) eine Zahl von 1 bis 50 ist oder für eine gemischte Alkylenoxygruppe der Formel -(O-A¹)ₐ-(O-A²)_{b}- steht, die statistisch oder blockweise angeordnet sein kann, und in der (a+b) eine Zahl von 1 bis 50 ist, (O-A¹) für eine Ethylenoxyeinheit und (O-A²) für eine Propylenoxyeinheit, für eine Butylenoxyeinheit, für eine Phenyloxiraneinheit, oder für Mischungen aus diesen stehen,
- R⁵: Wasserstoff ist,
- c: für eine Zahl von 1 bis 25, bevorzugt 1 bis 20, besonders bevorzugt 2 bis 15, insbesondere bevorzugt 4 bis 12, außerordentlich bevorzugt 6 bis 10 steht, und
- R⁴: für Wasserstoff steht.

Ein weiterer bevorzugter Gegenstand der Erfindung sind Ethanolamin-Glycerinether Kondensationsprodukte der Formel (I), **dadurch gekennzeichnet, dass** die Reste R¹, R² und R³ gleich sind und für eine Gruppe der Formel (IIc) stehen 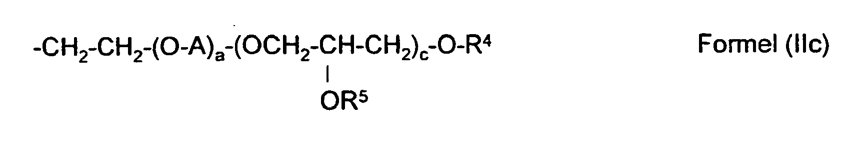 worin
- (O-A): für eine Alkylenoxygruppe, bevorzugt für eine Propylenoxygruppe, steht,
- a: für eine Zahl von 1 bis 50 steht,
- R⁵: Wasserstoff ist,
- c: für eine Zahl von 1 bis 25, bevorzugt 1 bis 20, besonders bevorzugt 2 bis 15, insbesondere bevorzugt 4 bis 12, außerordentlich bevorzugt 6 bis 10 steht, und
- R⁴: für Wasserstoff steht.

Ein weiterer bevorzugter Gegenstand der Erfindung sind Ethanolamin-Glycerinether Kondensationsprodukte der Formel (I), **dadurch gekennzeichnet, dass**
- R¹: für eine Alkylgruppe mit 1 bis 20, bevorzugt 3 bis 20 und besonders bevorzugt 4 bis 18 Kohlenstoffatomen, steht, und
- R² und R³: gleich sind und für eine Gruppe der Formel (IId) stehen 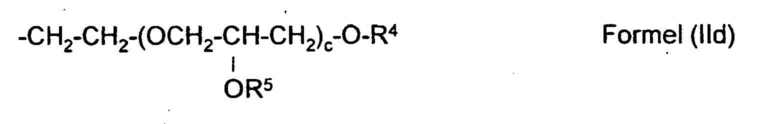
worin
- R⁵: Wasserstoff ist,
- c: für eine Zahl von 1 bis 25, bevorzugt 1 bis 20, besonders bevorzugt 2 bis 15, insbesondere bevorzugt 4 bis 12, außerordentlich bevorzugt 6 bis 10 steht, und
- R⁴: für Wasserstoff steht.

Ein weiterer bevorzugter Gegenstand der Erfindung sind Ethanoiamin-Glycerinether Kondensationsprodukte der Formel (I), **dadurch gekennzeichnet, dass**
- R¹: für eine Alkylgruppe mit 1 bis 10 Kohlenstoffatomen, bevorzugt für eine n-Butylgruppe, steht und
- R² und R³: gleich sind und für eine Gruppe der Formel (IId) stehen 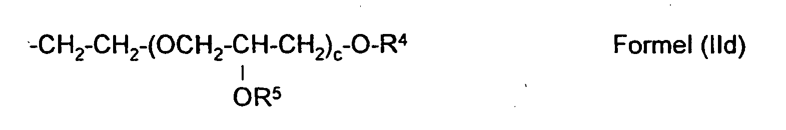
worin
- R⁵: Wasserstoff ist,
- c: für eine Zahl von 1 bis 25, bevorzugt 1 bis 20, besonders bevorzugt 2 bis 15, insbesondere bevorzugt 4 bis 12, außerordentlich bevorzugt 6 bis 10 steht, und
- R⁴: für Wasserstoff steht.

Die erfindungsgemäßen Ethanolamin-Glycerinether Kondensationsprodukte eignen sich in vorteilhafter Weise zur Herstellung von Dispersionen. Weiterer Gegenstand der vorliegenden Erfindung sind Dispersionen enthaltend einen oder mehrere der erfindungsgemäßen Ethanolamin-Glycerinether Kondensationsprodukte.

Die erfindungsgemäßen Ethanolamin-Glycerinether Kondensationsprodukte eignen sich zudem in vorteilhafter Weise als grenzflächenaktive Stoffe. Weiterer Gegenstand der vorliegenden Erfindung ist daher auch die Verwendung eines oder mehrerer der erfindungsgemäßen Ethanolamin-Glycerinether Kondensationsprodukte als grenzflächenaktive Stoffe.

In einer weiteren bevorzugten Ausführungsform der Erfindung werden die erfindungsgemäßen Ethanolamin-Glycerinether Kondensationsprodukte als grenzflächenaktive Stoffe in Dispersionen verwendet.

### Beispiele

Im Folgenden sind Herstellbeispiele von erfindungsgemäßen Glycerinethern beschrieben ohne die Erfindung auf diese einzuschränken.

### Allgemeine Herstellweise

### A) Polymerisation von Glycerin zu Oligoglycerinen bzw. Polyglycerinen

Die Polymerisation von Glycerin zu Oligoglycerinen bzw. Polyglycerinen erfolgt in bekannter Weise.

Hierzu wird Glycerin in Gegenwart von sauren Katalysatoren, beispielsweise HCl, H₂SO₄, Sulfonsäuren oder H₃PO₄ oder in Gegenwart von alkalischen Katalysatoren, wie Natriumhydroxid, Kaliumhydroxid, Alkalimetallalkoholaten, Alkalicarbonaten, Alkalibicarbonaten in einem Konzentrationsbereich von 0,1 bis 0,4 Gew.-% Katalysator in einer Rührapparatur mit Wasserabscheider und Stickstoffdurchleitung bei 200 bis 280°C, bevorzugt 240 bis 270°C, erhitzt. Unter Austrag von Kondensationswasser erfolgt innerhalb von 3 bis 14 Stunden die Bildung des Oligoglycerins bzw. Polyglycerins mit einem mittleren Kondensationsgrad von 2 bis 100, bevorzugt 3 bis 35 Glycerineinheiten. Aus der OH-Zahl lässt sich die mittlere Molmasse der Oligo- bzw. Polyglycerine berechnen.

Das Verhältnis des Kondensationsgrades n zur Kondensationszeit bei der Polymerisation von Glycerin zu Oligoglycerinen bzw. Polyglycerinen ist in Tabelle 1 dargestellt.

**Tabelle 1 Verhältnis von Kondensationsgrad n zu Kondensationszeit**

| Kondensationsgrad n | OH-Zahl [mg KOH/g] | Molare Masse [g/mol] | Kondensationszeit [Stunden] |
|---|---|---|---|
| 2,0 | 1352 | 166 | 3-4 |
| 3,0 | 1169 | 240 | 3-4 |
| 4,0 | 1072 | 314 | 4 - 5 |
| 5,0 | 1012 | 388 | 5 - 7 |
| 6,0 | 971 | 462 | 6-7 |
| 7,0 | 942 | 536 | 7 - 9 |
| 8,0 | 920 | 610 | 8 - 9 |
| 9,0 | 902 | 684 | 9 - 11 |
| 10 | 888 | 758 | 10 - 11 |
| 11 | 877 | 832 | 11 - 12 |
| 12 | 867 | 906 | 11 - 12 |
| 13 | 859 | 980 | 12-13 |
| 14 | 850 | 1056 | 12 - 13 |
| 15 | 844 | 1130 | 13 - 14 |

### B) Herstellung von Ethanolamin Glycerinethern

Zur Herstellung der erfindungsgemäßen Ethanolamin Glycerinether Kondensationsprodukte werden die oben genannten Mono-, Oligo- oder Polyglycerine bzw. die entsprechenden oxalkylierten Glycerine mit stickstoffhaltigen Mono- oder Polyolen und gegebenenfalls weiteren Comonomeren im gewünschten Mischungsverhältnis zusammengegeben. Nach Zugabe von 0,1-1,0 Mol-% (bevorzugt 0,28 Mol-%) eines basischen Katalysators (z.B. NaOH, KOH, Na₂CO₃, K₂CO₃, CaCO₃, Li₂CO₃, vorzugsweise NaOH, besonders bevorzugt 50 gew.-%ige wässrige NaOH) wird das Reaktionsgemisch unter starkem Rühren auf 220-290°C, bevorzugt 250-270°C, erhitzt und bei dieser Temperatur unter Auskreisung des entstehenden Wassers so lange kondensiert, bis der gewünschte Kondensationsgrad erreicht ist. Die Verfolgung des Reaktionsverlaufs erfolgt durch regelmäßige Bestimmung der OH-Zahl.

Die erfindungsgemäßen Verbindungen zeichnen sich durch ein ausgezeichnetes Dispergiervermögen und eine hohe Elektrolytstabilität aus. Zusätzlich bewirken die erfindungsgemäßen Glycerinether eine Verbesserung der Kompatibilität hydrophiler und hydrophober Komponenten und insbesondere eine Steigerung des Netz- und Absorptionsvermögens von Formulierungen, die diese Glycerinether enthalten.

### Beispiel 1 Cokondensation von Glycerin/Alkyldiethanolamin

Glycerin und Alkyldiethanolamin werden im gegebenen Molverhältnis zusammen mit 0,1 bis 1,0 Mol-% NaOH (bezogen auf eingesetzte Menge an Glycerin) in Form einer 50 Gew.-%igen wässrigen NaOH-Lösung vorgelegt und anschließend unter Rühren bei 220°C und leichter Stickstoffüberlagerung kondensiert, bis die errechnete Ziel-OH-Zahl in etwa erreicht ist (s. Tabelle 2). Die erhaltenen Produkte sind dunkelbraun und fest.

**Tabelle 2 Ethanolamin-Glycerinether Kondensationsprodukte mit Alkyldiethanolamin**

| Alkylkette | Molverhältnis Glycerin: Alkyldiethanolamin | Reaktionszeit | OH-Zahl Produkt | Kondensationsgrad* | mittlere Molmasse aus OH-Zahl |
|---|---|---|---|---|---|
| 2-Ethylhexyl | 10:1 | 19h | 701 mg KOH/g | 5.0 | 960 g/mol |
| 2-Ethylhexyl | 30:1 | 17 h | 732 mg KOH/g | 13 | 2130 g/mol |
| Cocos (C₈-C₁₈) | 10:1 | 11 h | 655 mg KOH/g | 4.4 | 925 g/mol |
| Cocos (C₈-C₁₈) | 30:1 | 12 h | 729 mg KOH/g | 20 | 3230 g/mol |

| | | | | | |
|---|---|---|---|---|---|
| * aus OH-Zahl errechneter Kondensationsgrad des Glycerins pro CH₂OH-Gruppe des Alkyldiethanolamins | | | | | |

### Beispiel 2 Cokondensation von Glycerin/Triethanolamin

Glycerin und Triethanolamin werden im gegebenen Molverhältnis zusammen mit 0,1-1,0 Mol-% NaOH (bezogen auf eingesetzte Menge an Glycerin) in Form einer 50 Gew.-%igen wässrigen NaOH-Lösung vorgelegt und unter Rühren bei 270°C und leichter Stickstoffüberlagerung kondensiert, bis die errechnete Ziel OH-Zahl in etwa erreicht ist (s. Tabelle 3). Die erhaltenen Produkte sind dunkelbraun und zähfließend bis fest.

**Tabelle 3 Ethanolamin-Glycerinether Kondensationsprodukte mit Triethanolamin**

| Molverhältnis Glycerin: Triethanolamin | Reaktionszeit | Reaktionstemperatur | OH-Zahl Produkt | Kondensationsgrad* | mittlere Molmasse aus OH-Zahl |
|---|---|---|---|---|---|
| 9:1 | 6 h | 270°C | 820 mg KOH/g | 3.3 | 883 g/mol |
| 15:1 | 9 h | 270°C | 795 mg KOH/g | 6.1 | 1500 g/mol |
| 30:1 | 14 h | 270°C | 781 mg KOH/g | 10.2 | 2410 g/mol |
| 45:1 | 18 h | 270°C | 770 mg KOH/g | 20.3 | 4660 g/mol |

| | | | | | |
|---|---|---|---|---|---|
| * aus OH-Zahl errechneter Kondensationsgrad des Glycerins pro CH₂OH-Gruppe des Triethanolamins | | | | | |

## Patentansprüche

1. Verwendung eines oder mehreres Ethanolamin-Glycerinether Kondensationsprodukte der Formel (I) 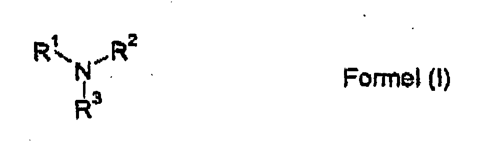 worin
R¹, R² und R³ jeweils unabhängig voneinander gleich oder verschieden sein können und
für Wasserstoff, für eine lineare oder verzweigte, gesättigte Alkylkette mit 1 bis 20, vorzugsweise 3 bis 20 und besonders bevorzugt 4 bis 18 Kohlenstoffatomen,
für eine lineare oder verzweigte ungesättigte Alkenylgruppe mit 2 bis 20, vorzugsweise 3 bis 20 und besonders bevorzugt 4 bis 18 Kohlenstoffatomen, für -CH₂-CH₂-OH oder
für einen Rest der Formel (II) 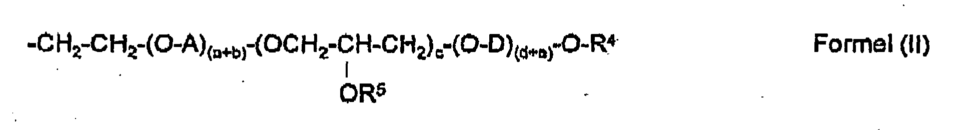 stehen,
worin
(O-A) für eine Alkylenoxygruppe ausgewählt aus Ethylenoxygruppe (EO), Propylenoxygruppe (PO), Butylenoxygruppe und/oder Phenyloxirangruppe,
a für eine Zahl von 0 bis 50, vorzugsweise 0 bis 20, besonders bevorzugt 0 bis 10,
b für eine Zahl von 0 bis 50, vorzugsweise 0 bis 20, besonders bevorzugt 0 bis 10,
R⁵ für Wasserstoff und/oder
für (O-A)ₐH, worin (O-A) für eine Alkylenoxygruppe ausgewählt aus Ethylenoxygruppe (EO), Propylenoxygruppe (PO), Butylenoxygruppe und/oder Phenyloxirangruppe und a für eine Zahl von 1 bis 30, stehen und/oder
für -(CR⁶R⁷)ₐ-Phenyl, wobei R⁶ und R⁷, die gleich oder verschieden sein können, für -H, für eine OH-Gruppe, für lineares oder verzweigtes (C₁-C₁₀)-Alkyl oder für lineares oder verzweigtes (C₂-C₃₀)-Alkenyl stehen und a für eine Zahl von 1 bis 10 steht, und/oder
für -(CR⁶R⁷)ₐ-Naphtyl, wobei R⁶ und R⁷, die gleich oder verschieden sein können, für -H, für eine OH-Gruppe, für lineares oder verzweigtes (C₁-C₁₀)-Alkyl oder für lineares oder verzweigtes (C₂-C₃₀)-Alkenyl stehen und a für eine Zahl von 1 bis 10 steht, und/oder für eine Gruppe der Formel (III) 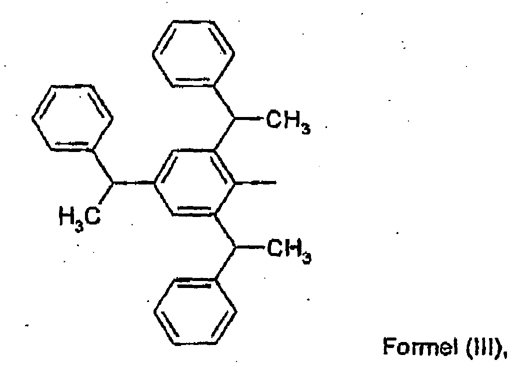 und/oder
für R⁸R⁹N-(CH₂)_{b}-, wobei R⁸ und R⁹, die gleich oder verschieden sein können, für -H, lineares oder verzweigtes (C₁-C₁₀)-Alkyl oder lineares oder verzweigtes (C₂-C₃₀)-Alkenyl stehen und b für eine Zahl von 1 bis 22 steht, und/oder
für HO-(CH₂)_{b}-, wobei b für eine Zahl von 1 bis 22 steht, und/oder für -SO₃⁻X⁺, -PO₃²⁻X⁺X'⁺ oder -CH₂COO⁻X⁺, wobei X⁺ und X'⁺ für H⁺, Li⁺, Na⁺, K⁺, Ca²⁺/2, Mg²⁺/2 oder N(R⁶)₄⁺ stehen und die R⁶ gleich oder verschieden sein können und für -H oder (C₁-C₁₀)-Alkyl, vorzugsweise (C₁-C₄)-Alkyl, stehen, und/oder
für eine Gruppe der Formel (IV) 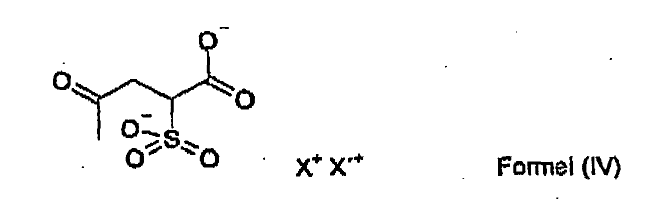 wobei X⁺ und X'⁺ für M⁺, Li⁺, Na⁺, K⁺, Ca²⁺/2, Mg²⁺/2 oder N(R⁸)₄⁺ stehen und die R⁸ gleich oder verschieden sein können und für -H oder (C₁-C₁₀)-Alkyl, vorzugsweise (C₁-C₄)-Alkyl, stehen, und/oder
für -C(R¹¹)₂-COO⁻X⁺, -CO-R¹²-COO⁻X⁺ oder -C(R¹¹)₂C(R¹¹)₂C(R¹¹)₂-N(R¹³)₂, wobei die R¹¹ gleich oder verschieden sein können und für -H und/oder -CH₃ stehen, R¹² für (C₁-C₁₀)-Alkylen oder (C₂-C₃₀)-Alkenylen steht, die R¹³ gleich oder verschieden sein können und für (C₁-C₁₀)-Alkyl oder (C₂-C₂₀)-Alkenyl stehen, X⁺ für H⁴, Li⁺, Na⁺, K⁺, Ca²⁺/2, Mg²⁺/2 oder N(R⁸)₄⁺ steht und die R⁸ gleich oder verschieden sein können und für -H oder (C₁-C₁₀)-Alkyl, vorzugsweise für (C₁-C₄)-Alkyl, stehen, und/oder
für -C(R¹⁴)₂C(R¹⁴)₂C(R¹⁴)₂-N((GO)_{z}H)₂, wobei die R¹⁴ gleich oder verschieden sein können und für -H und/oder -CH₃ stehen, G für -C₂H₄-, -C₃H₅- oder -C₄H₈- und z für eine Zahl von 1 bis 22 steht, und/oder für eine Gruppe der Formel (V) 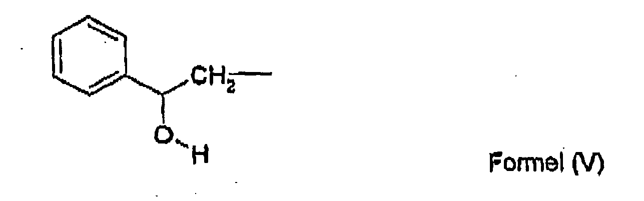 steht,
c für eine Zahl von 1 bis 25, vorzugsweise für eine Zahl von 1 bis 20, (O-D) für eine Alkylenoxygruppe ausgewählt aus Ethylenoxygruppe (EO), Propylenoxygruppe (PO), Butylenoxygruppe und/oder Phenyloxirangruppe,
d für eine Zahl von 0 bis 50, vorzugsweise 0 bis 20, besonders bevorzugt 0 bis 10,
e für eine Zahl von 0 bis 50, vorzugsweise 0 bis 20, besonders bevorzugt 0 bis 10, stehen und
R⁴ die Bedeutung von R⁵ haben kann, mit der Maßgabe, dass mindestens einer der Reste R¹, R² und R³ ein Rest der Formel (II) ist und mindestens ein weiterer der Reste R¹, R² und R³ ausgewählt ist aus -CH₂-CH₂-OH und Resten der Formel (II) als grenzflächenaktive Stoffe.

2. Verwendung gemäß Anspruch 1, **dadurch gekennzeichnet, dass** in den Ethanolamin-Glycerinether Kondensationsprodukten mindestens zwei der Reste R¹, R² und R³ Reste der Formel (II) sind.

3. Verwendung gemäß Anspruch 1 oder 2, **dadurch gekennzeichnet, dass** in den Ethanolamin-Glycerinether Kondensationsprodukten zwei der Reste R¹, R² und R³ Reste der Formel (II) sind.

4. Verwendung gemäß Anspruch 3, **dadurch gekennzeichnet, dass** in den Ethanolamin-Glycerinether Kondensationsprodukten der dritte der Reste R¹, R² und R³ für eine lineare oder verzweigte, gesättigte Alkylkette mit 1 bis 20, vorzugsweise 3 bis 20 und besonders bevorzugt 4 bis 18 Kohlenstoffatomen oder für eine lineare oder verzweigte ungesättigte Alkenylgruppe mit 2 bis 20, vorzugsweise 3 bis 20 und besonders bevorzugt 4 bis 18 Kohlenstoffatomen steht.

5. Verwendung gemäß Anspruch 4, **dadurch gekennzeichnet, dass** in den Ethanolamin-Glycerinether Kondensationsprodukten der dritte der Reste R¹, R² und R³ für eine lineare oder verzweigte, gesättigte Alkylkette mit 1 bis 20, vorzugsweise 3 bis 20 und besonders bevorzugt 4 bis 18 Kohlenstoffatomen steht.

6. Verwendung gemäß Anspruch 1 oder 2, **dadurch gekennzeichnet, dass** in den Ethanolamin-Glycerinether Kondensationsprodukten alle drei Reste R¹, R² und R³ ausgewählt sind aus -CH₂-H₂-OH und Resten der Formel (II).

7. Verwendung gemäß Anspruch 6, **dadurch gekennzeichnet, dass** in den Ethanolamin-Glycerinether Kondensationsprodukten alle drei Reste R¹, R² und R³ Reste der Formel (II) sind.

8. Verwendung gemäß einem oder mehreren der Ansprüche 1 bis 7, **dadurch gekennzeichnet, dass** in den Ethanolamin-Glycerinether Kondensationsprodukten (O-A) und (O-D) jeweils für eine einheitliche Alkylenoxygruppe, (a+b) für eine Zahl von 0 bis 20 und (d+e) für eine Zahl von 0 bis 20 stehen.

9. Verwendung gemäß einem oder mehreren der Ansprüche 1 bis 7, **dadurch gekennzeichnet, dass** in den Ethanolamin-Glycerinether Kondensationsprodukten (O-A)_{(a+b)} und (O-D)_{(d+a)} für gemischte Alkylenoxygruppen der Formel -(O-A¹)ₐ-(O-A²)_{b}- bzw. -(O-D¹)_{d}-(O-D²)ₐ-, die statistisch oder blockweise angeordnet sein können, stehen und in denen a und b jeweils eine Zahl von 1 bis 30 bedeuten sowie d und e jeweils eine Zahl von 1 bis 20 bedeuten, (O-A¹) und (O-D¹) für eine Ethylenoxyeinheit, und (O-A²) und (O-D²) für eine Propylenoxyelnheit, für eine Butylenoxyeinheit, für eine Phenyloxiraneinheit, oder für Mischungen aus diesen stehen.

10. Verwendung gemäß einem oder mehreren der Ansprüche 1, 2 und 6 bis 8, **dadurch gekennzeichnet, dass** in den Ethanolamin-Glycerinether Kondensationsprodukten die Reste R¹, R² und R³ gleich sind und für eine Gruppe der Formel (IIa) stehen, 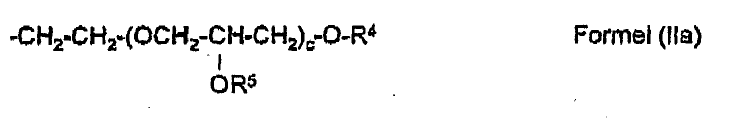 worin
R⁵ Wasserstoff ist,
c für eine Zahl von 1 bis 25, bevorzugt 1 bis 20, besonders bevorzugt 2 bis 15, insbesondere bevorzugt 4 bis 12, außerordentlich bevorzugt 6 bis 10 steht, und
R⁴ Wasserstoff ist.

11. Verwendung gemäß einem oder mehreren der Ansprüche 1, 2 und 6 bis 8, **dadurch gekennzeichnet, dass** in dem Ethanolamin-Glycerinether Kondensationsprodukten die Reste R¹_{,} R² und R³ gleich sind und für eine Gruppe der Formel (IIa) stehen, 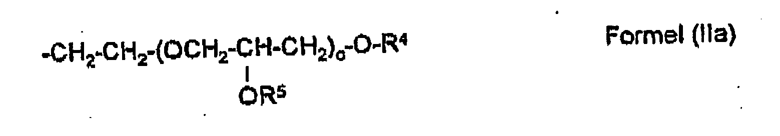 worin
R⁵ Wasserstoff ist,
c für eine Zahl von 1 bis 25, bevorzugt 1 bis 20, besonders bevorzugt 2 bis 15, insbesondere bevorzugt 4 bis 12, außerordentlich bevorzugt 6 bis 10 steht, und
R⁴ für eine Gruppe der Formel (III) 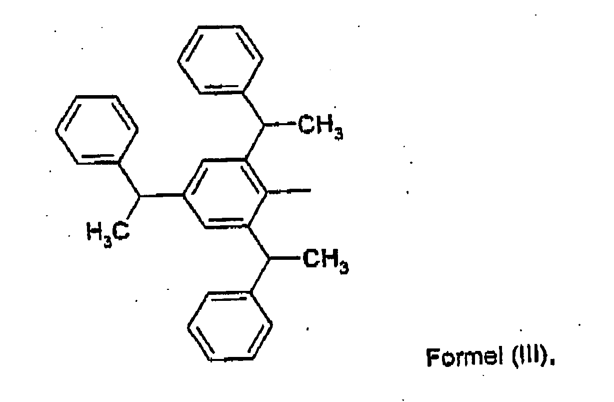 steht.

12. Verwendung gemäß einem oder mehreren der Ansprüche 1, 2, 6 und 7, **dadurch gekennzeichnet, dass** in den Ethanolamin-Glycerinether Kondensationsprodukten die Reste R¹, R² und R³ gleich sind und für eine Gruppe der Formel (IIb) stehen 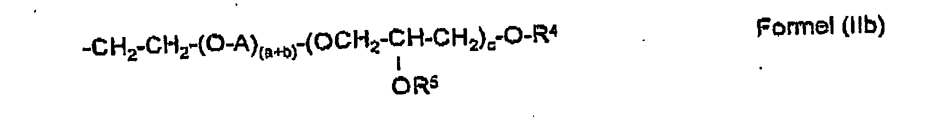 worin
(O-A)_{(a+b)} für eine einheitliche Alkylenoxygruppe steht und (a+b) eine Zahl von 1 bis 50 ist oder für eine gemischte Alkylenoxygruppe der Formel -(O-A')ₐ-(O-A²)_{b}- steht, die statistisch oder blockweise angeordnet sein kann und in der (a+b) eine Zahl von 1 bis 50 ist, (O-A¹) für eine Ethylenoxyelnheit und (O-A²) für eine Propylenoxyeinheit, für eine Butylenoxyeinheit, für eine Phonyloxiraneinheit oder für Mischungen aus diesen stehen,
R⁵ Wasserstoff ist,
c für eine Zahl von 1 bis 25, bevorzugt 1 bis 20, besonders bevorzugt 2 bis 15, insbesondere bevorzugt 4 bis 12, außerordentlich bevorzugt 6 bis 10 steht, und
R⁴ für Wasserstoff steht.

13. Verwendung gemäß einem oder mehreren der Ansprüche 1, 2, 6, 7 und 12, **dadurch gekennzeichnet, dass** in den Ethanolamin-Glycerinether Kondensationsprodukten die Reste R¹, R² und R³ gleich sind und für eine Gruppe der Formel (IIc) stehen 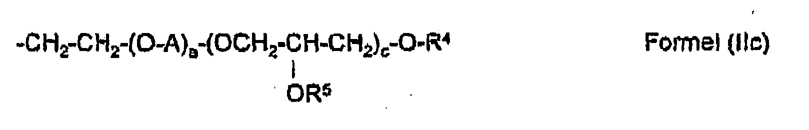 worin (O-A) für eine Alkylenoxygruppe, bevorzugt für eine Propylenoxygruppe, steht,
a für eine Zahl von 1 bis 50 steht,
R⁵ Wasserstoff ist,
c für eine Zahl von 1 bis 25, bevorzugt 1 bis 20, besonders bevorzugt 2 bis 15, insbesondere bevorzugt 4 bis 12, außerordentlich bevorzugt 6 bis 10 steht, und
R⁴ für Wasserstoff steht.

14. Verwendung gemäß einem oder mehreren der Ansprüche 1 bis 5 und 8, **dadurch gekennzeichnet, dass** in den Ethanolamin-Glycerinether Kondensationsprodukten
R¹ für eine Alkylgruppe mit 1 bis 20, vorzugsweise 3 bis 20 und besonders bevorzugt 4 bis 18 Kohlenstoffatomen, steht und
R² und R³ gleich sind und für eine Gruppe der Formel (IId) stehen 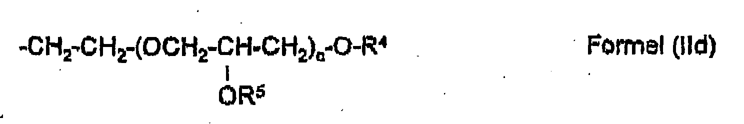 worin
R⁵ Wasserstoff ist,
c für eine Zahl von 1 bis 25, bevorzugt 1 bis 20, besonders bevorzugt 2 bis 15, insbesondere bevorzugt 4 bis 12, außerordentlich bevorzugt 6 bis 10 steht, und
R⁴ für Wasserstoff steht.

15. Verwendung gemäß einem oder mehreren der Ansprüche 1 bis 5, 8 und 14, **dadurch gekennzeichnet, dass** in den Ethanolamin-Glycerinether Kondensationsprodukten
R¹ für eine Alkylgruppe mit 1 bis 10 Kohlenstoffatomen, bevorzugt für eine n-Butylgruppe, steht und
R² und R³ gleich sind und für eine Gruppe der Formel (IId) stehen 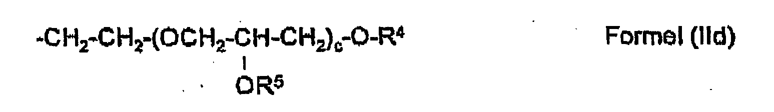 worin
R⁵ Wasserstoff ist,
c für eine Zahl von 1 bis 25, bevorzugt 1 bis 20, besonders bevorzugt 2 bis 15, insbesondere bevorzugt 4 bis 12, außerordentlich bevorzugt 6 bis 10 steht, und
R⁴ für Wasserstoff steht.

16. Verwendung gemäß einem oder mehreren der Ansprüche 1 bis 15 in Dispersionen.

17. Ethanolamin-Glycerinether Kondensationsprodukt der Formel (I) 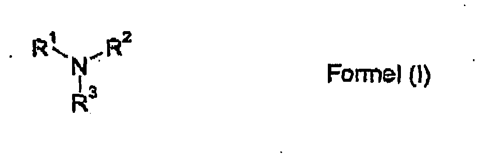 worin
R¹, R² und R³ jeweils unabhängig voneinander gleich oder verschieden sein können und für Wasserstoff,
für eine lineare oder verzweigtes, gesättigte Alkylkette mit 1 bis 20, vorzugsweise 3 bis 20 und besonders bevorzugt 4 bis 18 Kohlenstoffatomen,
für eine lineare oder verzweigte ungesättigte Alkenylgruppe mit 2 bis 20, vorzugsweise 3 bis 20 und besonders bevorzugt 4 bis 18 Kohlenstoffatomen, für -CH₂-CH₂-OH oder
für einen Rest der Formel (II) 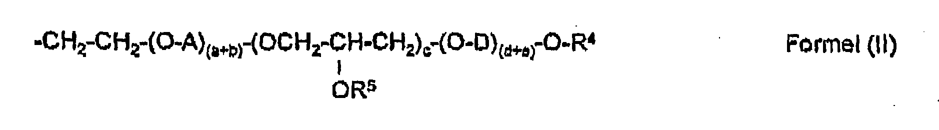 stehen,
worin
(O-A) für eine Alkylenoxygruppe ausgewählt aus Ethylenoxygruppe (EO), Propylenoxygruppe (PO), Butylenoxygruppe und/oder Phenyloxirangruppe,
a für eine Zahl von 0 bis 50, vorzugeweise 0 bis 20, besonders bevorzugt 0 bis 10.
b für eine Zahl von 0 bis 50, vorzugsweise 0 bis 20, besonders bevorzugt 0 bis 10,
R⁵ für Wasserstoff und/oder
für (O-A)_{B}H, worin (O-A) für eine Alkylenoxygruppe ausgewählt aus Ethylenoxygruppe (EO), Propylenoxygruppe (PO), Butylenoxygruppe und/oder Phenyloxirangruppe und a für eine Zahl von 1 bis 30, stehen und/oder
für -(CR⁶R⁷)ₐ-Phenyl, wobei R⁵ und R⁷, die gleich oder verschieden sein können, für -H, für eine OH-Gruppe, für lineares oder verzweigtes (C₁-C₁₀)-Alkyl oder für lineares oder verzweigtes (C₂-C₃₀)-Alkenyl stehen und a für eine Zahl von 1 bis 10 steht, und/oder
für -(CR⁶R⁷)ₐ-Naphtyl, wobei R⁶ und R⁷, die gleich oder verschieden sein können, für -H, für eine OH-Gruppe, für lineares oder verzweigtes (C₁-C₁₀)-Alkyl oder für lineares oder verzweigtes (C₂-C₃₀)-Alkenyl stehen und a für eine Zahl von 1 bis 10 steht, und/oder für eine Gruppe der Formel (III) 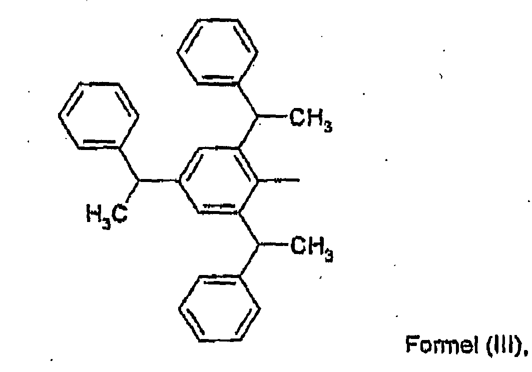 und/oder
für R⁸R⁹N-(CH₂)_{b}-, wobei R⁸ und R⁹, die gleich oder verschieden sein können, für -H, lineares oder verzweigtes (C₁-C₁₀)-Alkyl oder lineares oder verzweigtes (C₂-C₃₀)-Alkenyl stehen und b für eine Zahl von 1 bis 22 steht,
und/oder
für HO-(CH₂)_{b}-, wobei b für eine Zahl von 1 bis 22 steht, und/oder für -SO₃⁻X⁺, -PO₃²⁻X⁺X⁻⁺ oder -CH₂COO⁻X⁺, wobei X⁺ und X'⁺ für H⁺, Li⁺, Na⁺, K⁺, Ca²⁺/2, Mg²⁺/2 oder N(R⁸)₄⁺ stehen und die R⁸ gleich oder verschieden sein können und für -H oder (C₁-C₁₀)-Alkyl, vorzugsweise (C₁-C₄)-Alkyl, stehen, und/oder
für eine Gruppe der Formel (IV) 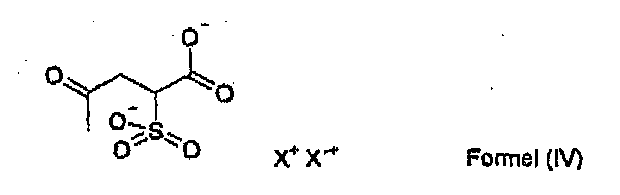 wobei X⁺ und X'⁺ für H⁺, Li⁺, Na⁺, K⁺, Ca²⁺/2, Mg²⁺/2 oder N(R⁸)₄⁺ stehen und die R⁸ gleich oder verschieden sein können und für -H oder (C₁-C₁₀)-Alkyl, vorzugsweise (C₁-C₄)-Alkyl, stehen, und/oder
für -C(R¹¹)₂-COO-X⁺, -CO-R¹²-COO⁻X⁺ oder -C(R¹¹)₂C(R¹¹)₂C(R¹¹)₂-N(R¹³)₂, wobei die R¹¹ gleich oder verschieden sein können und für -H und/oder -CH₃ stehen, R¹² für (C₁-C₁₀)-Alkylen oder (C₂-C₃₀)-Alkenylen steht, die R¹³ gleich oder verschieden sein können und für (C₁-C₁₀)-Alkyl oder (C₂-C₃₀)-Alkenyl stehen, X⁺ für H⁺, Li⁺, Na⁺, K⁺, Ca²⁺/2, Mg²⁺/2 oder N(R⁸)₄⁺ steht und die R⁸ gleich oder verschieben sein können und für -H oder (C₁-C₁₀)-Alkyl, vorzugsweise für (C₁-C₄)-Alkyl, stehen, und/oder
für -C(R¹⁴)₂C(R¹⁴)₂C(R¹⁴)₂-N((GO)_{z}H)₂, wobei die R¹⁴ gleich oder verschieden, sein können und für -H und/oder -CH₃ stehen, G für -C₂H₄-, -C₃H₈- oder -C₄H₈- und z für eine Zahl von 1 bis 22 steht, und/oder für eine Gruppe der Formel (V) 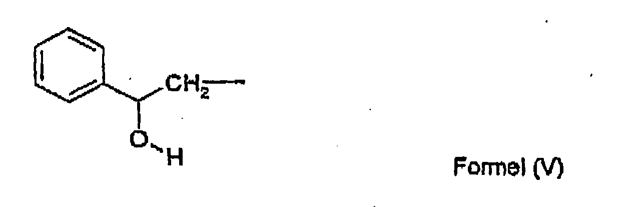 steht,
c für eine Zahl von 1 bis 25, vorzugsweise für eine Zahl von 1 bis 20, (O-D) für eine Alkylenoxygruppe ausgewählt aus Ethylenoxygruppe (EO), Propylenoxygruppe (PO), Butylenoxygruppe und/oder Phenyloxirangruppe,
d für eine Zahl von 0 bis 50, vorzugsweise 0 bis 20, besonders bevorzugt 0 bis 10,
e für eine Zahl von 0 bis 50, vorzugsweise 0 bis 20, besonders bevorzugt 0 bis 10, stehen und
R⁴ die Bedeutung von R⁵ haben kann,
mit der Maßgabe, dass mindestens zwei der Reste R¹, R² und R³ Reste der Formel (II) sind und mindestens ein weiterer der Reste R¹, R² und R³ ausgewählt ist aus -CH₂-CH₂-OH und Resten der Formel (II).

18. Ethanolamin-Glycerinether Kondensationsprodukt gemäß Anspruch 17,
**dadurch gekennzeichnet, dass** zwei der Reste R¹, R² und R³ Reste der Formel (II) sind.

19. Ethanolamin-Glycerinether Kondensationsprodukt gemäß Anspruch 18, **dadurch gekennzeichnet, dass** der dritte der Reste R¹, R² und R³ für eine lineare oder verzweigte, gesättigte Alkylkette mit 1 bis 20, vorzugsweise 3 bis 20 und besonders bevorzugt 4 bis 18 Kohlenstoffatomen oder für eine lineare oder verzweigte ungesättigte Alkenylgruppe mit 2 bis 20, vorzugsweise 3 bis 20 und besonders bevorzugt 4 bis 18 Kohlenstoffatomen steht.

20. Ethanolamin-Glycerlnether Kondensationsprodukt gemäß Anspruch 19, **dadurch gekennzeichnet, dass** der dritte der Reste R¹, R² und R³ für eine lineare oder verzweigte, gesättigte Alkylkette mit 1 bis 20, vorzugsweise 3 bis 20 und besonders bevorzugt 4 bis 18 Kohlenstoffatomen steht.

21. Ethanolamin-Glycerinether Kondensationsprodukt gemäß Anspruch 17,
**dadurch gekennzeichnet, dass** alle drei Reste R¹, R² und R³ ausgewählt sind aus -CH₂-H₂-OH und Resten der Formel (II).

22. Ethanolamin-Glycerinether Kondensationsprodukt gemäß Anspruch 21, **dadurch gekennzeichnet, dass** alle drei Reste R¹, R² und R³ Reste der Formel (II) sind.

23. Ethanolamin-Glycerinether Kondensationsprodukt gemäß einem oder mehreren der Ansprüche 17 bis 22, **dadurch gekennzeichnet, dass** (O-A) und (O-D) jeweils für eine einheitliche Alkylenoxygruppe, (a+b) für eine Zahl von 0 bis 20 und (d+e) für eine Zahl von 0 bis 20 stehen.

24. Ethanolamin-Glycerinether Kondensationsprodukt gemäß einem oder mehreren der Ansprüche 17 bis 22, **dadurch gekennzeichnet, dass** (O-A)_{(a+b)} und (O-D)_{(d+e)} für gemischte Alkylenoxygruppen der Formel -(O-A¹)ₐ-(O-A²)_{b}- bzw. -(O-D¹)_{d}-(O-D²)ₑ-, die statistisch oder blockweise angeordnet sein können, stehen und in denen a und b jeweils eine Zahl von 1 bis 30 bedeuten sowie d und e jeweils eine Zahl von 1 bis 20 bedeuten, (O-A¹) und (O-D¹) für eine Ethylenoxyeinheit, und (O-A²) und (O-D²) für eine Propylenoxyeinheit, für eine Butylenoxyelnheit, für eine Phenyloxiraneinheit, oder für Mischungen aus diesen stehen.

25. Ethanolamin-Glycerinether Kondensationsprodukt gemäß einem oder mehreren der Ansprüche 17 und 21 bis 23, **dadurch gekennzeichnet, dass** die Reste R¹, R² und R³ gleich sind und für eine Gruppe der Formel (IIa) stehen, 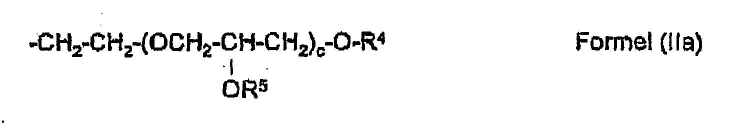 worin
R⁵ Wasserstoff ist,
c für eine Zahl von 1 bis 25, bevorzugt 1 bis 20, besonders bevorzugt 2 bis 15, insbesondere bevorzugt 4 bis 12, außetordentlich bevorzugt 6 bis 10 steht, und
R⁴ Wasserstoff ist.

26. Ethanolamin-Glycerinether Kondensationsprodukt gemäß einem oder mehreren der Ansprüche 17 und 21 bis 23, **dadurch gekennzeichnet, dass** die Reste R¹, R² und R³ gleich sind und für eine Gruppe der Formal (IIa) stehen, 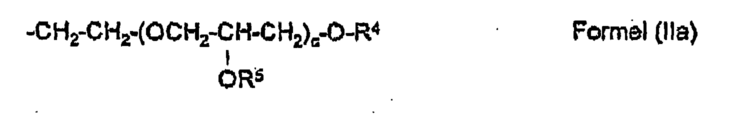 worin
R⁵ Wasserstoff ist,
c für eine Zahl von 1 bis 25, bevorzugt 1 bis 20, besonders bevorzugt 2 bis 15, Insbesondere bevorzugt 4 bis 12, außerordentlich bevorzugt 6 bis 10 steht, und
R⁴ für eine Gruppe der Formel (III) 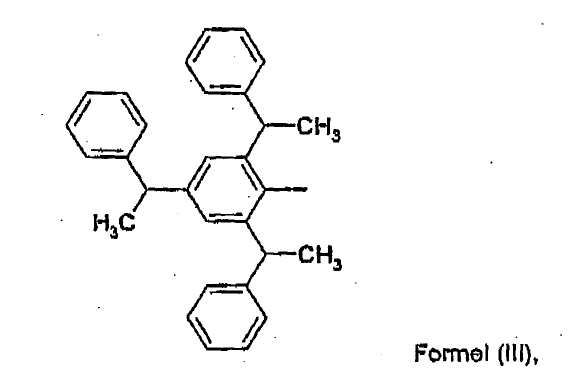 steht.

27. Ethanolamin-Glycerinether Kondensationsprodukt gemäß einem oder mehreren der Ansprüche 17, 21 und 22, **dadurch gekennzeichnet, dass** die Reste R¹, R² und R³ gleich sind und für eine Gruppe der Formel (IIb) stehen 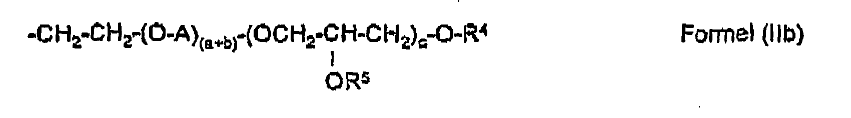 worin
(O-A)_{(a+b)} für eine einheitliche Alkylenoxygruppe steht und (a+b) eine Zahl von 1 bis 50 ist oder für eine gemischte Alkylenoxygruppe der Formel -(O-A¹)ₐ-(O-A²)_{b}- steht, die statistisch oder blockweise angeordnet sein kann und in der (a+b) eine Zahl von 1 bis 50 ist, (O-A¹) für eine Ethylenoxyeinheit und (O-A²) für eine Propylenoxyeinheit, für eine Butylenoxyeinheit, für eine Phenyloxiraneinheit oder für Mischungen aus diesen stehen,
R⁵ Wasserstoff ist,
c für eine Zahl von 1 bis 25, bevorzugt 1 bis 20, besonders bevorzugt 2 bis 15, insbesondere bevorzugt 4 bis 12, außerordentlich bevorzugt 6 bis 10 steht, und
R⁴ für Wasserstoff steht.

28. Ethanolamin-Glycerinether Kondensationsprodukt gemäß einem oder mehreren der Ansprüche 17, 21, 22 und 27, **dadurch gekennzeichnet, dass** die Reste R¹, R² und R³ gleich sind und für eine Gruppe der Formel (IIc) stehen 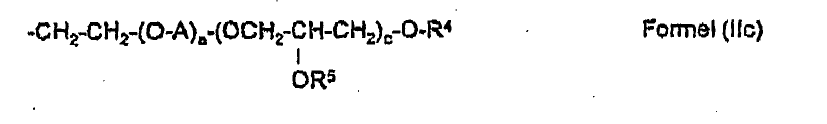 worin
(O-A) für eine Alkylenoxygruppe, bevorzugt für eine Propylenoxygruppe, steht, a für eine Zahl von 1 bis 50 steht,
R⁵ Wasserstoff ist,
c für eine Zahl von 1 bis 25, bevorzugt 1 bis 20, besonders bevorzugt 2 bis 15, insbesondere bevorzugt 4 bis 12, außerordentlich bevorzugt 6 bis 10 steht, und
R⁴ für Wasserstoff steht

29. Ethanolamin-Glycerinether Kondensationsprodukt gemäß einem oder mehreren der Ansprüche 17 bis 20 und 23, **dadurch gekennzeichnet, dass**
R¹ für eine Alkylgruppe mit 1 bis 20, vorzugsweise 3 bis 20 und besonders bevorzugt 4 bis 18 Kohlenstoffatomen, steht und
R² und R³ gleich sind und für eine Gruppe der Formel (IId) stehen 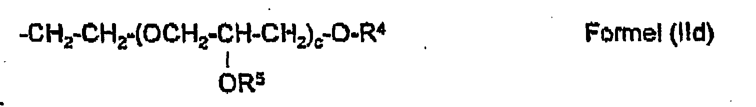 worin
R⁵ Wasserstoff ist,
c für eine Zahl von 1 bis 25, bevorzugt 1 bis 20, besonders bevorzugt 2 bis 15, insbesondere bevorzugt 4 bis 12, außerordentlich bevorzugt 6 bis 10 steht, und
R⁴ für Wasserstoff steht.

30. Ethanolamin-Glycerinether Kondensationsprodukt gemäß einem oder mehreren der Ansprüche 17 bis 20, 23 und 29, **dadurch gekennzeichnet, dass**
R¹ für eine Alkylgruppe mit 1 bis 10 Kohlenstoflatomen, bevorzugt für eine n-Butylgruppe, steht und
R² und R³ gleich sind und für eine Gruppe der Formel (IId) stehen 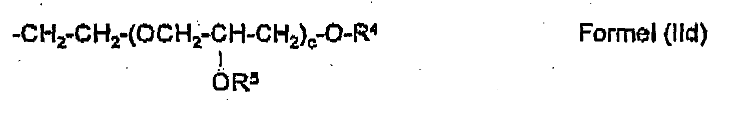 worin
R⁵ Wasserstoff ist,
c für eine Zahl von 1 bis 25, bevorzugt 1 bis 20, besonders bevorzugt 2 bis 15, insbesondere bevorzugt 4 bis 12, außerordentlich bevorzugt 6 bis 10 steht, und
R⁴ für Wasserstoff steht.

31. Dispersion enthaltend einen oder mehrere Ethanolamin-Glycerinether Kondensationsprodukte gemäß einem oder mehreren der Ansprüche 17 bis 30.

## Claims

1. The use of one or more ethanolamine glyceryl ether condensation products of the formula (I) 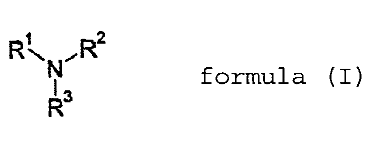 in which
R¹, R² and R³, in each case independently of one another, may be identical or different and are hydrogen,
are a linear or branched, saturated alkyl chain having 1 to 20, preferably 3 to 20 and particularly preferably 4 to 18, carbon atoms,
are a linear or branched unsaturated alkenyl group having 2 to 20, preferably 3 to 20 and particularly preferably 4 to 18, carbon atoms, are -CH₂-CH₂-OH or
are a radical of the formula (II) 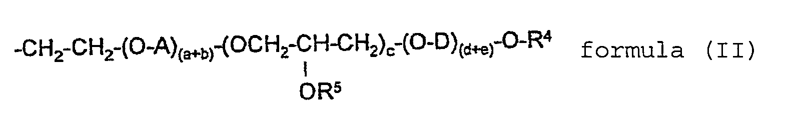 in which
(O-A)is an alkyleneoxy group selected from ethyleneoxy group (EO), propyleneoxy group (PO), butyleneoxy group and/or phenyloxirane group,
a is a number from 0 to 50, preferably 0 to 20, particularly preferably 0 to 10,
b is a number from 0 to 50, preferably 0 to 20, particularly preferably 0 to 10,
R⁵ is hydrogen and/or
is (O-A)ₐH, in which (O-A) is an alkyleneoxy group selected from ethyleneoxy group (EO), propyleneoxy group (PO), butyleneoxy group and/or phenyloxirane group and a is a number from 1 to 30, and/or
is -(CR⁶R⁷)ₐ-phenyl, where R⁶ and R⁷, which may be identical or different, are -H, are an OH group, are linear or branched (C₁-C₁₀)-alkyl or are linear or branched (C₂-C₃₀)-alkenyl and a is a number from 1 to 10, and/or
is -(CR⁶R⁷)ₐ-naphthyl, where R⁶ and R⁷, which may be identical or different, are -H, are an OH group, are linear or branched (C₁-C₁₀)-alkyl or are linear or branched (C₂-C₃₀)-alkenyl and a is a number from 1 to 10, and/or
is a group of the formula (III) 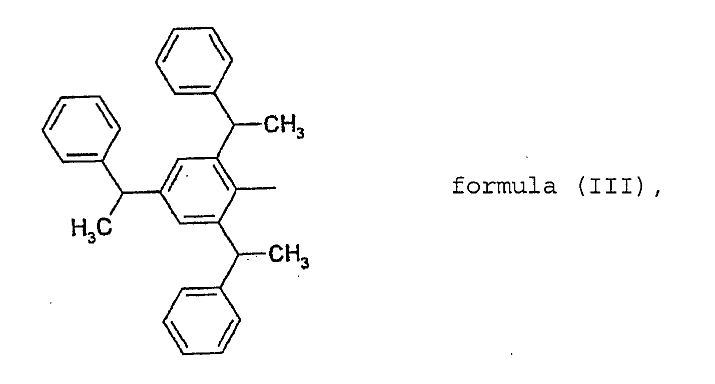 is R⁸R⁹N-(CH₂)_{b}-, where R⁸ and R⁹, which may be identical or different, are -H, linear or branched (C₁-C₁₀)-alkyl or linear or branched (C₂-C₃₀)-alkenyl and b is a number from 1 to 22, and/or
is HO-(CH₂)_{b}-, where b is a number from 1 to 22, and/or
is -SO₃⁻X⁺, -PO₃²⁻X⁺X'⁺ or -CH₂COO⁻X⁺, where X⁺ and X'⁺ are H⁺, Li⁺, Na⁺, K⁺, Ca²⁺/2, Mg²⁺/2 or N(R⁸)₄⁺ and the R⁸ may be identical or different and are -H or (C₁-C₁₀)-alkyl, preferably (C₁-C₄)-alkyl, and/or
is a group of the formula (IV) 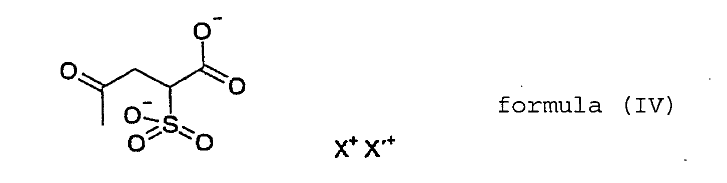 where X⁺ and X'⁺ are H⁺, Li⁺, Na⁺, K⁺, Ca²⁺/2, Mg²⁺/2 or N(R⁸)₄⁺, and the R⁸ may be identical or different and are -H or (C₁-C₁₀)-alkyl, preferably (C₁-C₄)-alkyl, and/or
is -C(R¹¹)₂-COO⁻X⁺, -CO-R¹²-COO⁻X⁺ or -C(R¹¹)₂C(R¹¹)₂C(R¹¹)₂-N(R¹³)₂, where the R¹¹ may be identical or different and are -H and/or CH₃, R¹² is (C₁-C₁₀)-alkylene or (C₂-C₃₀) -alkenylene, the R¹³ may be identical or different and are (C₁-C₁₀)-alkyl or (C₂-C₃₀)-alkenyl, X⁺ is H⁺, Li⁺, Na⁺, K⁺, Ca²⁺/2, Mg²⁺/2 or N(R⁸)₄⁺, and the R⁸ may be identical or different and are -H or (C₁-C₁₀)-alkyl, preferably (C₁-C₄)-alkyl, and/or
is -C(R¹⁴)₂C(R¹⁴)₂C(R¹⁴)₂-N((GO)_{z}H)₂, where the R¹⁴ may be identical or different and are -H and/or -CH₃, G is -C₂H₄-, -C₃H₆- or -C₄H₈- and z is a number from 1 to 22, and/or
is a group of the formula (V) 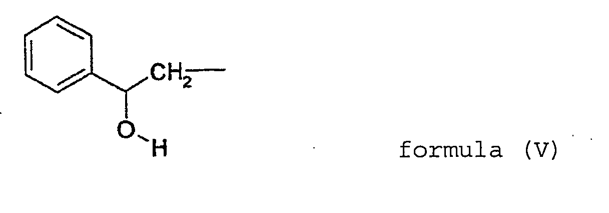
c is a number from 1 to 25, preferably a number from 1 to 20, (O-D) is an alkyleneoxy group selected from ethyleneoxy group (EO), propyleneoxy group (PO), butyleneoxy group and/or phenyloxirane group,
d is a number from 0 to 50, preferably 0 to 20, particularly preferably 0 to 10,
e is a number from 0 to 50, preferably 0 to 20, particularly preferably 0 to 10, and
R⁴ can have the meaning of R⁵,
with the proviso that at least one of the radicals R¹, R² and R³ is a radical of the formula (II) and at least one of the other radicals R¹, R² and R³ is selected from -CH₂-CH₂-OH and radicals of the formula (II) as interface-active substances.

2. The use as claimed in claim 1, wherein, in the ethanolamine glyceryl ether condensation products, at least two of the radicals R¹, R² and R³ are radicals of the formula (II).

3. The use as claimed in claim 1 or 2, wherein, in the ethanolamine glyceryl ether condensation products, two of the radicals R¹, R² and R³ are radicals of the formula (II).

4. The use as claimed in claim 3, wherein, in the ethanolamine glyceryl ether condensation products, the third of the radicals R¹, R² and R³ is a linear or branched, saturated alkyl chain having 1 to 20, preferably 3 to 20 and particularly preferably 4 to 18, carbon atoms or is a linear or branched unsaturated alkenyl group having 2 to 20, preferably 3 to 20 and particularly preferably 4 to 18, carbon atoms.

5. The use as claimed in claim 4, wherein, in the ethanolamine glyceryl ether condensation products, the third of the radicals R¹, R² and R³ is a linear or branched, saturated alkyl chain having 1 to 20, preferably 3 to 20 and particularly preferably 4 to 18, carbon atoms.

6. The use as claimed in claim 1 or 2, wherein, in the ethanolamine glyceryl ether condensation products, all three radicals R¹, R² and R³ are selected from -CH₂-H₂-OH and radicals of the formula (II).

7. The use as claimed in claim 6, wherein, in the ethanolamine glyceryl ether condensation products, all three radicals R¹, R² and R³ are radicals of the formula (II).

8. The use as claimed in one or more of claims 1 to 7, wherein, in the ethanolamine glyceryl ether condensation products, (O-A) and (O-D) are in each case a uniform alkyleneoxy group, (a+b) is a number from 0 to 20 and (d+e) is a number from 0 to 20.

9. The use as claimed in one or more of claims 1 to 7, wherein, in the ethanolamine glyceryl ether condensation products, (O-A)_{(a+b)} and (O-D)_{(d+e)} are mixed alkyleneoxy groups of the formula -(O-A¹)ₐ-(O-A²)_{b}- or -(O-D¹)_{d}-(O-D²)ₑ-, which may be arranged randomly or blockwise, and in which a and b are in each case a number from 1 to 30, and d and e are in each case a number from 1 to 20, (O-A¹) and (O-D¹) are an ethyleneoxy unit, and (O-A²) and (O-D²) are a propyleneoxy unit, a butyleneoxy unit, a phenyloxirane unit, or mixtures of these.

10. The use as claimed in one or more of claims 1, 2 and 6 to 8, wherein, in the ethanolamine glyceryl ether condensation products, the radicals R¹, R² and R³ are identical and are a group of the formula (IIa), 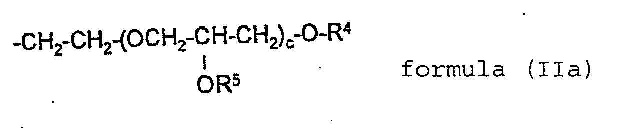 in which
R⁵ is hydrogen,
c is a number from 1 to 25, preferably 1 to 20, particularly preferably 2 to 15, especially preferably 4 to 12, extraordinarily preferably 6 to 10, and
R⁴ is hydrogen.

11. The use as claimed in one or more of claims 1, 2 and 6 to 8, wherein, in the ethanolamine glyceryl ether condensation products, the radicals R¹, R² and R³ are identical and are a group of the formula (IIa), 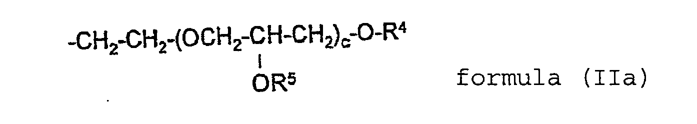 in which
R⁵ is hydrogen,
c is a number from 1 to 25, preferably 1 to 20, particularly preferably 2 to 15, especially preferably 4 to 12, extraordinarily preferably 6 to 10, and
R⁴ is a group of the formula (III)

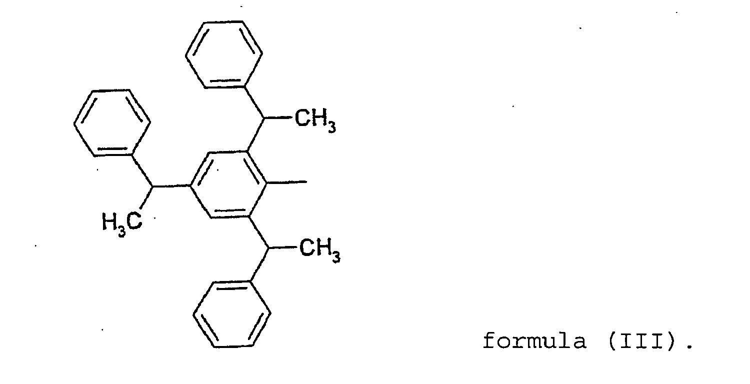

12. The use as claimed in one or more of claims 1, 2, 6 and 7, wherein, in the ethanolamine glyceryl ether condensation products, the radicals R¹, R² and R³ are identical and are a group of the formula (IIb), 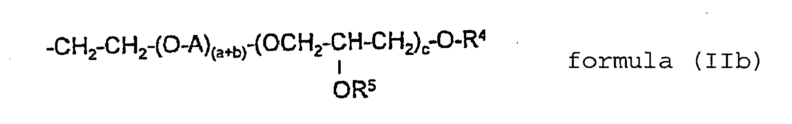 in which
(O-A)_{(a+b)} is a uniform alkyleneoxy group and (a+b) is a number from 1 to 50 or is a mixed alkyleneoxy group of the formula -(O-A¹)ₐ-(O-A²)_{b}-, which can be arranged randomly or blockwise and in which (a+b) is a number from 1 to 50, (O-A¹) is an ethyleneoxy unit and (O-A²) is a propyleneoxy unit, a butyleneoxy unit, a phenyloxirane unit or mixtures of these,
R⁵ is hydrogen,
c is a number from 1 to 25, preferably 1 to 20, particularly preferably 2 to 15, especially preferably 4 to 12, extraordinarily preferably 6 to 10, and
R⁴ is hydrogen.

13. The use as claimed in one or more of claims 1, 2, 6, 7 and 12, wherein, in the ethanolamine glyceryl ether condensation products, the radicals R¹, R² and R³ are identical and are a group of the formula (IIc), 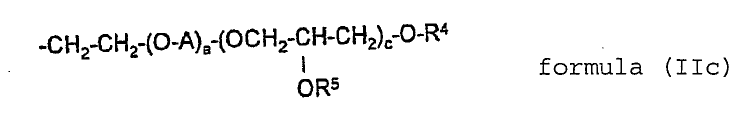 in which
(O-A) is an alkyleneoxy group, preferably a propyleneoxy group,
a is a number from 1 to 50,
R⁵ is hydrogen,
c is a number from 1 to 25, preferably 1 to 20, particularly preferably 2 to 15, especially preferably 4 to 12, extraordinarily preferably 6 to 10, and
R⁴ is hydrogen.

14. The use as claimed in one or more of claims 1 to 5 and 8, wherein, in the ethanolamine glyceryl ether condensation products,
R¹ is an alkyl group having 1 to 20, preferably 3 to 20 and particularly preferably 4 to 18, carbon atoms, and
R² and R³ are identical and are a group of the formula (IId) 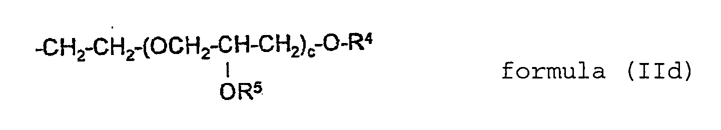 in which
R⁵ is hydrogen,
c is a number from 1 to 25, preferably 1 to 20, particularly preferably 2 to 15, especially preferably 4 to 12, extraordinarily preferably 6 to 10, and
R⁴ is hydrogen.

15. The use as claimed in one or more of claims 1 to 5, 8 and 14, wherein, in the ethanolamine glyceryl ether condensation products,
R¹ is an alkyl group having 1 to 10 carbon atoms, preferably an n-butyl group, and
R² and R³ are identical and are a group of the formula (IId) 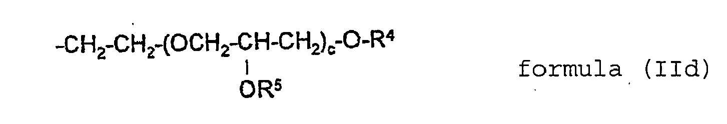 in which
R⁵ is hydrogen,
c is a number from 1 to 25, preferably 1 to 20, particularly preferably 2 to 15, especially preferably 4 to 12, extraordinarily preferably 6 to 10, and
R⁴ is hydrogen.

16. The use as claimed in one or more of claims 1 to 15 in dispersions.

17. An ethanolamine glyceryl ether condensation product of the formula (I) 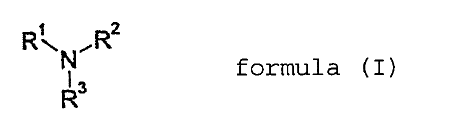 in which
R¹, R² and R³, in each case independently of one another, may be identical or different and are hydrogen,
are a linear or branched, saturated alkyl chain having 1 to 20, preferably 3 to 20 and particularly preferably 4 to 18, carbon atoms,
are a linear or branched unsaturated alkenyl group having 2 to 20, preferably 3 to 20 and particularly preferably 4 to 18, carbon atoms, are -CH₂-CH₂-OH or
are a radical of the formula (II) 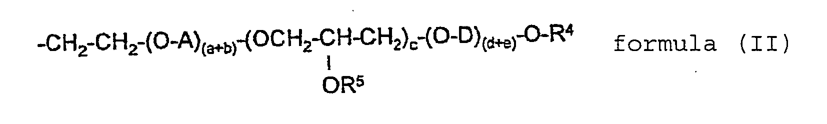 in which
(O-A) is an alkyleneoxy group selected from ethyleneoxy group (EO), propyleneoxy group (PO), butyleneoxy group and/or phenyloxirane group,
a is a number from 0 to 50, preferably 0 to 20, particularly preferably 0 to 10,
b is a number from 0 to 50, preferably 0 to 20, particularly preferably 0 to 10,
R⁵ is hydrogen and/or
is (O-A)ₐH, in which (O-A) is an alkyleneoxy group selected from ethyleneoxy group (EO), propyleneoxy group (PO), butyleneoxy group and/or phenyloxirane group and a is a number from 1 to 30, and/or
is - (CR⁶R⁷)ₐ-phenyl, where R⁶ and R⁷, which may be identical or different, are -H, are an OH group, are linear or branched (C₁-C₁₀)-alkyl or are linear or branched (C₂-C₃₀)-alkenyl and a is a number from 1 to 10, and/or
is -(CR⁶R⁷)ₐ-naphthyl, where R⁶ and R⁷, which may be identical or different, are -H, are an OH group, are linear or branched (C₁-C₁₀)-alkyl or are linear or branched (C₂-C₃₀)-alkenyl and a is a number from 1 to 10, and/or
is a group of the formula (III) 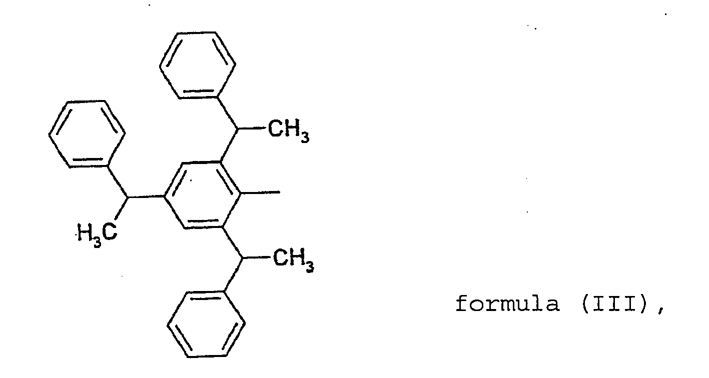 is R⁸R⁹N-(CH₂)_{b}-, where R⁸ and R⁹, which may be identical or different, are -H, linear or branched (C₁-C₁₀)-alkyl or linear or branched (C₂-C₃₀) -alkenyl and b is a number from 1 to 22, and/or
is HO-(CH₂)_{b}-, where b is a number from 1 to 22, and/or
is -SO₃⁻X⁺, -PO₃²⁻X⁺X'⁺ or -CH₂COO⁻X⁺, where X⁺ and X'⁺ are H⁺, Li⁺, Na⁺, K⁺, Ca²⁺/2, Mg²⁺/2 or N(R⁸)₄⁺ and the R⁸ may be identical or different and are -H or (C₁-C₁₀)-alkyl, preferably (C₁-C₄)-alkyl, and/or
is a group of the formula (IV) 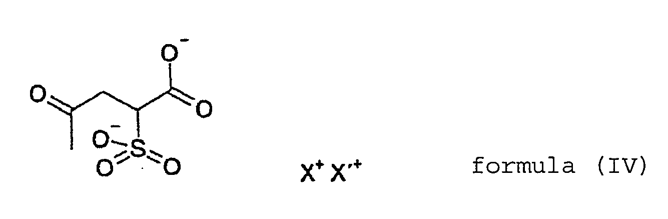 where X⁺ and X'⁺ are H⁺, Li⁺, Na⁺, K⁺, Ca²⁺/2, Mg²⁺/2 or N(R⁸)₄⁺, and the R⁸ may be identical or different and are -H or (C₁-C₁₀)-alkyl, preferably (C₁-C₄)-alkyl, and/or
is -C(R¹¹)₂-COO⁻X⁺, -CO-R¹²-COO⁻X⁺ or -C(R¹¹)₂C(R¹¹)₂C(R¹¹)₂-N(R¹³)₂, where the R¹¹ may be identical or different and are -H and/or CH₃, R¹² is (C₁-C₁₀)-alkylene or (C₂-C₃₀)-alkenylene, the R¹³ may be identical or different and are (C₁-C₁₀)-alkyl or (C₂-C₃₀)-alkenyl, X⁺ is H⁺, Li⁺, Na⁺, K⁺, Ca²⁺/2, Mg²⁺/2 or N(R⁸)₄⁺, and the R⁸ may be identical or different and are -H or (C₁-C₁₀)-alkyl, preferably (C₁-C₄)-alkyl, and/or
is -C(R¹⁴)₂C(R¹⁴)₂C(R¹⁴)₂-N((GO)_{z}H)₂, where the R¹⁴ may be identical or different and are -H and/or -CH₃, G is -C₂H₄-, -C₃H₆- or -C₄H₈- and z is a number from 1 to 22, and/or
is a group of the formula (V) 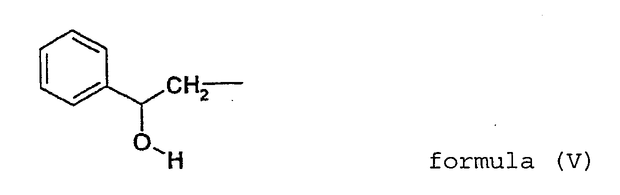
c is a number from 1 to 25, preferably a number from 1 to 20, (O-D) is an alkyleneoxy group selected from ethyleneoxy group (EO), propyleneoxy group (PO), butyleneoxy group and/or phenyloxirane group,
d is a number from 0 to 50, preferably 0 to 20, particularly preferably 0 to 10,
e is a number from 0 to 50, preferably 0 to 20, particularly preferably 0 to 10, and
R⁴ can have the meaning of R⁵,
with the proviso that at least two of the radicals R¹, R² and R³ are radicals of the formula (II) and at least one of the other radicals R¹, R² and R³ is selected from -CH₂-CH₂-OH and radicals of the formula (II).

18. An ethanolamine glyceryl ether condensation product as claimed in claim 17, wherein two of the radicals R¹, R² and R³ are radicals of the formula (II).

19. An ethanolamine glyceryl ether condensation product as claimed in claim 18, wherein the third of the radicals R¹, R² and R³ is a linear or branched, saturated alkyl chain having 1 to 20, preferably 3 to 20 and particularly preferably 4 to 18, carbon atoms or is a linear or branched unsaturated alkenyl group having 2 to 20, preferably 3 to 20 and particularly preferably 4 to 18, carbon atoms.

20. An ethanolamine glyceryl ether condensation product as claimed in claim 19, wherein the third of the radicals R¹, R² and R³ is a linear or branched, saturated alkyl chain having 1 to 20, preferably 3 to 20 and particularly preferably 4 to 18, carbon atoms.

21. An ethanolamine glyceryl ether condensation product as claimed in claim 17, wherein all three radicals R¹, R² and R³ are selected from -CH₂-H₂-OH and radicals of the formula (II).

22. An ethanolamine glyceryl ether condensation product as claimed in claim 21, wherein all three radicals R¹, R² and R³ are radicals of the formula (II).

23. An ethanolamine glyceryl ether condensation product as claimed in one or more of claims 17 to 22, wherein (O-A) and (O-D) are in each case a uniform alkyleneoxy group, (a+b) is a number from 0 to 20 and (d+e) is a number from 0 to 20.

24. An ethanolamine glyceryl ether condensation product as claimed in one or more of claims 17 to 22, wherein (O-A)_{(a+b)} and (O-D)_{(d+e)} are mixed alkyleneoxy groups of the formula -(O-A¹)ₐ-(O-A²)_{b}- or -(O-D¹)_{d}-(O-D²)ₑ-, which may be arranged randomly or blockwise, and in which a and b are in each case a number from 1 to 30, and d and e are in each case a number from 1 to 20, (O-A¹) and (O-D¹) are an ethyleneoxy unit, and (O-A²) and (O-D²) are a propyleneoxy unit, a butyleneoxy unit, a phenyloxirane unit, or mixtures of these.

25. An ethanolamine glyceryl ether condensation product as claimed in one or more of claims 17, and 21 to 23, wherein the radicals R¹, R² and R³ are identical and are a group of the formula (IIa), 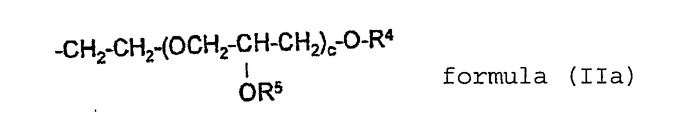 in which
R⁵ is hydrogen,
c is a number from 1 to 25, preferably 1 to 20, particularly preferably 2 to 15, especially preferably 4 to 12, extraordinarily preferably 6 to 10, and
R⁴ is hydrogen.

26. An ethanolamine glyceryl ether condensation product as claimed in one or more of claims 17, and 21 to 23, wherein the radicals R¹, R² and R³ are identical and are a group of the formula (IIa), 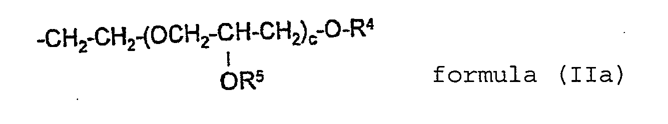 in which
R⁵ is hydrogen,
c is a number from 1 to 25, preferably 1 to 20, particularly preferably 2 to 15, especially preferably 4 to 12, extraordinarily preferably 6 to 10, and
R⁴ is a group of the formula (III)

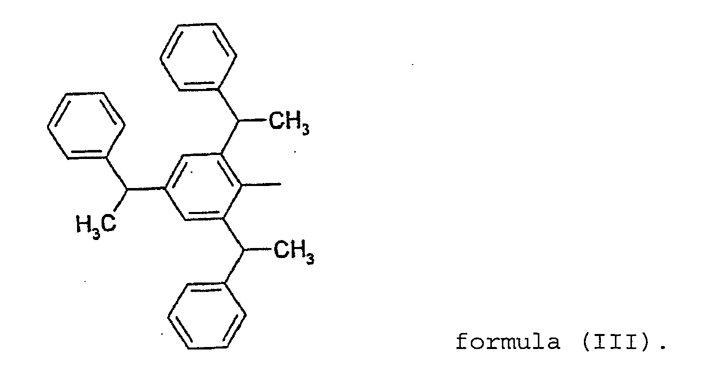

27. An ethanolamine glyceryl ether condensation product as claimed in one or more of claims 17, 21 and 22, wherein the radicals R¹, R² and R³ are identical and are a group of the formula (IIb) 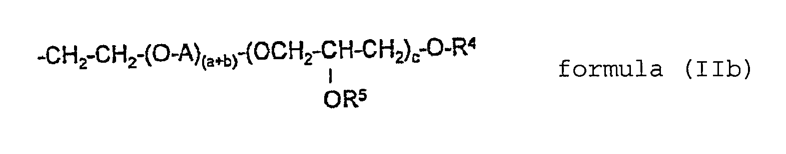 in which
(O-A)_{(a+b)} is a uniform alkyleneoxy group and (a+b) is a number from 1 to 50 or is a mixed alkyleneoxy group of the formula -(O-A¹)ₐ-(O-A²)_{b}-, which can be arranged randomly or blockwise and in which (a+b) is a number from 1 to 50, (O-A¹) is an ethyleneoxy unit and (O-A²) is a propyleneoxy unit, a butyleneoxy unit, a phenyloxirane unit or mixtures of these,
R⁵ is hydrogen,
c is a number from 1 to 25, preferably 1 to 20, particularly preferably 2 to 15, especially preferably 4 to 12, extraordinarily preferably 6 to 10, and
R⁴ is hydrogen.

28. An ethanolamine glyceryl ether condensation product as claimed in one or more of claims 17, 21, 22 and 27, wherein the radicals R¹, R² and R³ are identical and are a group of the formula (IIc) 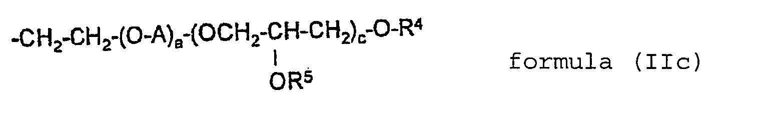 in which
(O-A) is an alkyleneoxy group, preferably a propyleneoxy group,
a is a number from 1 to 50,
R⁵ is hydrogen,
c is a number from 1 to 25, preferably 1 to 20, particularly preferably 2 to 15, especially preferably 4 to 12, extraordinarily preferably 6 to 10, and
R⁴ is hydrogen.

29. An ethanolamine glyceryl ether condensation product as claimed in one or more of claims 17 to 20 and 23, wherein
R¹ is an alkyl group having 1 to 20, preferably 3 to 20 and particularly preferably 4 to 18, carbon atoms, and
R² and R³ are identical and are a group of the formula (IId) 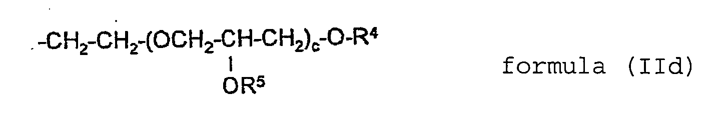 in which
R⁵ is hydrogen,
c is a number from 1 to 25, preferably 1 to 20, particularly preferably 2 to 15, especially preferably 4 to 12, extraordinarily preferably 6 to 10, and
R⁴ is hydrogen.

30. An ethanolamine glyceryl ether condensation product as claimed in one or more of claims 17 to 20, 23 and 29, wherein
R¹ is an alkyl group having 1 to 10 carbon atoms, preferably an n-butyl group, and
R² and R³ are identical and are a group of the formula (IId) 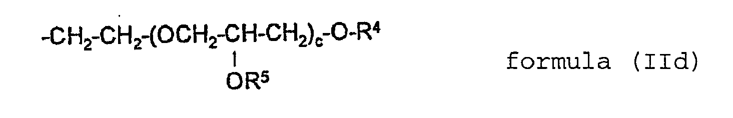 in which
R⁵ is hydrogen,
c is a number from 1 to 25, preferably 1 to 20, particularly preferably 2 to 15, especially preferably 4 to 12, extraordinarily preferably 6 to 10, and
R⁴ is hydrogen.

31. A dispersion comprising one or more ethanolamine glyceryl ether condensation products as claimed in one or more of claims 17 to 30.

## Revendications

1. Utilisation d'un ou de plusieurs produits de condensation d'éthanolamine-glycéroléther de formule (I) 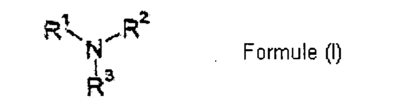 où
R¹, R² et R³ peuvent être identiques ou différents, à chaque fois indépendamment l'un de l'autre, et représentent hydrogène,
une chaîne alkyle linéaire ou ramifiée, saturée, comprenant 1 à 20, de préférence 3 à 20 et de manière particulièrement préférée 4 à 18 atomes de carbone,
un groupe alcényle linéaire ou ramifié, insaturé, comprenant 2 à 20, de préférence 3 à 20 et de manière particulièrement préférée 4 à 18 atomes de carbone,
-CH₂-CH₂-OH ou
un radical de formule (II) 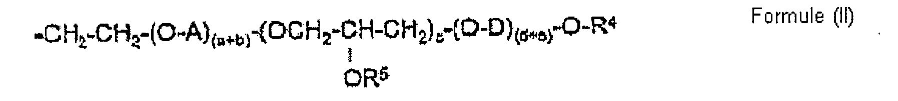 où
(O-A) représente un groupe d'alkylène-oxy choisi parmi le groupe d'éthylène-oxy (OE), le groupe de propylène-oxy (OP), le groupe de butylène-oxy et/ou le groupe de phényloxirane,
a représente un nombre de 0 à 50, de préférence de 0 à 20, de manière particulièrement préférée de 0 à 10,
b représente un nombre de 0 à 50, de préférence de 0 à 20, de manière particulièrement préférée de 0 à 10,
R⁵ représente hydrogène et/ou
(O-A)ₐH, où (O-A) représente un groupe d'alkylèneoxy choisi parmi le groupe d'éthylène-oxy (OE), le groupe de propylène-oxy (OP), le groupe de butylène-oxy et/ou le groupe de phényloxirane et a représente un nombre de 1 à 30 et/ou
-(CR⁶R⁷)ₐ-phényle, où R⁶ et R⁷, qui peuvent être identiques ou différents, représentent -H, un groupe OH, un groupe (C₁-C₁₀)-alkyle linéaire ou ramifié ou un groupe (C₂-C₃₀)-alcényle linéaire ou ramifié et a représente un nombre de 1 à 10, et/ou -(CR⁶R⁷)ₐ-naphtyle, où R⁶ et R⁷, qui peuvent être identiques ou différents, représentent -H, un groupe OH, un groupe (C₁-C₁₀)-alkyle linéaire ou ramifié ou un groupe (C₂-C₃₀)-alcényle linéaire ou ramifié et a représente un nombre de 1 à 10, et/ou un groupe de formule (III) 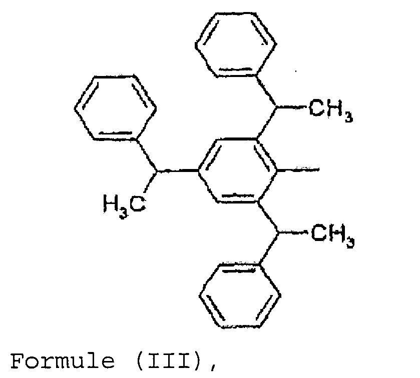 R⁸R⁹N- (CH₂)_{b}-, où R⁸ et R⁹, qui peuvent être identiques ou différents, représentent -H, un groupe (C₁-C₁₀)-alkyle linéaire ou ramifié ou un groupe (C₂-C₃₀)-alcényle linéaire ou ramifié et b représente un nombre de 1 à 22, et/ou
HO-(CH₂)_{b}-, où b représente un nombre de 1 à 22, et/ou
-SO₃⁻X⁺, -PO₃²⁻X⁺X'⁺ ou -CH₂COO⁻X⁺, où X⁺ et X'⁺ représentent H⁺, Li⁺, Na⁺, K⁺, Ca²⁺/2, Mg²⁺/2 ou N(R⁸)₄⁺ et les radicaux R⁸ peuvent être identiques ou différents et représentent -H ou (C₁-C₁₀)-alkyle, de préférence (C₁-C₄)-alkyle, et/ou un groupe de formule (IV) 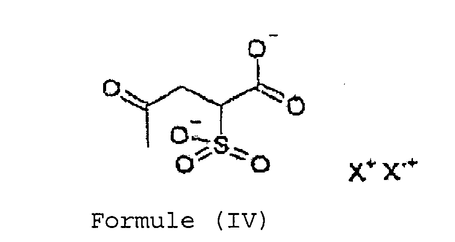 où X⁺ et X'⁺ représentent H⁺, Li⁺, Na⁺, K⁺, Ca²⁺/2, Mg²⁺/2 ou N(R⁸)₄⁺ et les radicaux R⁸ peuvent être identiques ou différents et représentent -H ou (C₁-C₁₀)-alkyle, de préférence (C₁-C₄)-alkyle, et/ou
-C(R¹¹)₂-COO⁻X⁺, -CO-R¹²-COO⁻X⁺ ou -C(R¹¹)₂C(R¹¹)₂C(R¹¹)₂-N(R¹³)₂, où les radicaux R¹¹ peuvent être identiques ou différents et représentent -H et/ou -CH₃, R¹² représente (C₁-C₁₀)-alkylène ou (C₂-C₃₀)-alcénylène, les radicaux R¹³ peuvent être identiques ou différents et représentent (C₁-C₁₀)-alkyle ou (C₂-C₃₀)-alcényle, X⁺ représente H⁺, Li⁺, Na⁺, K⁺, Ca²⁺/2, Mg²⁺/2 ou N(R⁸)₄⁺ et les radicaux R⁸ peuvent être identiques ou différents et représentent -H ou (C₁-C₁₀)-alkyle, de préférence (C₁-C₄)-alkyle, et/ou
-C(R¹⁴)₂C(R¹⁴)₂C(R¹⁴)₂-N((GO)_{z}H)₂, où les radicaux R¹⁴ peuvent être identiques ou différents et représentent -H et/ou -CH₃, G représente -C₂H₄-, -C₃H₆- ou -C₄H₈- et z représente un nombre de 1 à 22, et/ou
un groupe de formule (V) 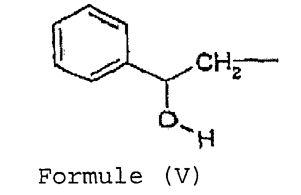
c représente un nombre de 1 à 25, de préférence un nombre de 1 à 20,
(O-D) représente un groupe d'alkylène-oxy choisi parmi le groupe d'éthylène-oxy (OE), le groupe de propylène-oxy (OP), le groupe de butylène-oxy et/ou le groupe de phényloxirane,
d représente un nombre de 0 à 50, de préférence de 0 à 20, de manière particulièrement préférée de 0 à 10,
e représente un nombre de 0 à 50, de préférence de 0 à 20, de manière particulièrement préférée de 0 à 10, et
R⁴ peut présenter la signification de R⁵,
à condition qu'au moins un des radicaux R¹, R² et R³ représente un radical de formule (II) et qu'au moins un autre des radicaux R¹, R² et R³ soit choisi parmi -CH₂-CH₂-OH et les radicaux de formule (II) comme substances tensioactives.

2. Utilisation selon la revendication 1,
**caractérisée en ce que** dans les produits de condensation d'éthanolamine-glycéroléther, au moins deux des radicaux R¹, R² et R³ sont des radicaux de formule (II).

3. Utilisation selon la revendication 1 ou 2,
**caractérisée en ce que** dans les produits de condensation d'éthanolamine-glycéroléther, deux des radicaux R¹, R² et R³ sont des radicaux de formule (II).

4. Utilisation selon la revendication 3,
**caractérisée en ce que** dans les produits de condensation d'éthanolamine-glycéroléther, le troisième des radicaux R¹, R² et R³ représente une chaîne alkyle linéaire ou ramifiée, saturée, comprenant 1 à 20, de préférence 3 à 20 et de manière particulièrement préférée 4 à 18 atomes de carbone ou un groupe alcényle linéaire ou ramifié, insaturé comprenant 2 à 20, de préférence 3 à 20 et de manière particulièrement préférée 4 à 18 atomes de carbone.

5. Utilisation selon la revendication 4,
**caractérisée en ce que** dans les produits de condensation d'éthanolamine-glycéroléther, le troisième des radicaux R¹, R² et R³ représente une chaîne alkyle linéaire ou ramifiée, saturée, comprenant 1 à 20, de préférence 3 à 20 et de manière particulièrement préférée 4 à 18 atomes de carbone.

6. Utilisation selon la revendication 1 ou 2,
**caractérisée en ce que** dans les produits de condensation d'éthanolamine-glycéroléther, les trois radicaux R¹, R² et R³ sont choisis parmi -CH₂-H₂-OH et les radicaux de formule (II).

7. Utilisation selon la revendication 6,
**caractérisée en ce que** dans les produits de condensation d'éthanolamine-glycéroléther, les trois radicaux R¹, R² et R³ sont des radicaux de formule (II).

8. Utilisation selon l'une ou plusieurs des revendications 1 à 7, **caractérisée en ce que,** dans les produits de condensation d'éthanolamine-glycéroléther, (O-A) et (O-D) représentent à chaque fois un groupe d'alkylène-oxy uniforme, (a+b) représente un nombre de 0 à 20 et (d+e) représente un nombre de 0 à 20.

9. Utilisation selon l'une ou plusieurs des revendications 1 à 7, **caractérisée en ce que** dans les produits de condensation d'éthanolamine-glycérol, (O-A)_{(a+b)} et (O-D)_{(d+e)} représentent des groupes d'alkylène-oxy mixtes de formule -(O-A¹)ₐ-(O-A²)_{b}- ou -(O-D¹)_{d}-(O-D²)ₑ-, qui peuvent être disposés de manière aléatoire ou par blocs et dans lesquels a et b signifient à chaque fois un nombre de 1 à 30 et d et e signifient à chaque fois un nombre de 1 à 20, (O-A¹) et (O-D¹) représentent une unité d'éthylène-oxy et (O-A²) et (O-D²) représentent une unité de propylène-oxy, une unité de butylène-oxy ou une unité de phényloxirane, ou des mélanges de ceux-ci.

10. Utilisation selon l'une ou plusieurs des revendications 1, 2 et 6 à 8, **caractérisée en ce que** dans les produits de condensation d'éthanolamine-glycéroléther, les radicaux R¹, R² et R³ sont identiques et représentent un groupe de formule (IIa) 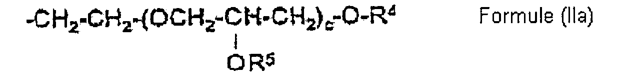 où
R⁵ représente hydrogène,
c représente un nombre de 1 à 25, de préférence de 1 à 20, de manière particulièrement préférée de 2 à 15, en particulier de préférence de 4 à 12, de manière extrêmement préférée de 6 à 10, et
R⁴ représente hydrogène.

11. Utilisation selon l'une ou plusieurs des revendications 1, 2 et 6 à 8, **caractérisée en ce que** dans les produits de condensation d'éthanolamine-glycéroléther, les radicaux R¹, R² et R³ sont identiques et représentent un groupe de formule (IIa) 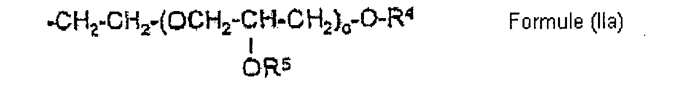 où
R⁵ représente hydrogène,
c représente un nombre de 1 à 25, de préférence de 1 à 20, de manière particulièrement préférée de 2 à 15, en particulier de préférence de 4 à 12, de manière extrêmement préférée de 6 à 10, et
R⁴ représente un groupe de formule (III)

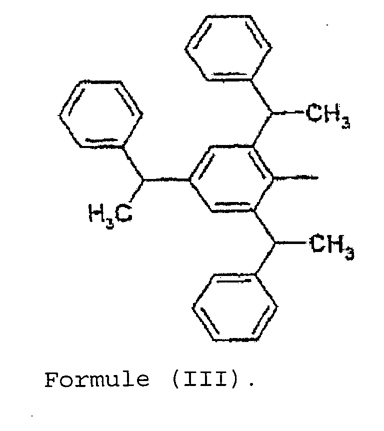

12. Utilisation selon l'une ou plusieurs des revendications 1, 2, 6 et 7, **caractérisée en ce que** dans les produits de condensation d'éthanolamine-glycéroléther, les radicaux R¹, R² et R³ sont identiques et représentent un groupe de formule (IIb) 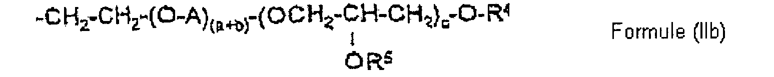 où
(O-A)_{(a+b)} représente un groupe d'alkylène-oxy uniforme et (a+b) représente un nombre de 1 à 50 ou un groupe d'alkylène-oxy mixte de formule -(O-A¹)ₐ-(O-A²)_{b}-, qui peut être disposé de manière aléatoire ou par blocs et où (a+b) représente un nombre de 1 à 50, (O-A¹) représente une unité d'éthylène-oxy et (O-A²) représente une unité de propylène-oxy, une unité de butylène-oxy, une unité de phényloxirane, ou les mélanges de ceux-ci,
R⁵ représente hydrogène,
c représente un nombre de 1 à 25, de préférence de 1 à 20, de manière particulièrement préférée de 2 à 15, en particulier de préférence de 4 à 12, de manière extrêmement préférée de 6 à 10, et
R⁴ représente hydrogène.

13. Utilisation selon l'une ou plusieurs des revendications 1, 2, 6, 7 et 12, **caractérisée en ce que** dans les produis de condensation d'éthanolamine-glycéroléther, les radicaux R¹, R² et R³ sont identiques et représentent un groupe de formule (IIc) 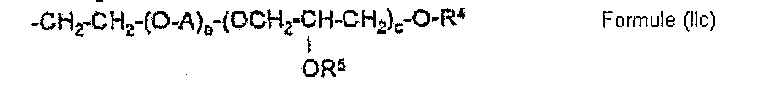 où
(O-A) représente un groupe d'alkylène-oxy, de préférence un groupe de propylène-oxy,
a représente un nombre de 1 à 50,
R⁵ représente hydrogène,
c représente un nombre de 1 à 25, de préférence de 1 à 20, de manière particulièrement préférée de 2 à 15, en particulier de préférence de 4 à 12, de manière extrêmement préférée de 6 à 10, et
R⁴ représente hydrogène.

14. Utilisation selon l'une ou plusieurs des revendications 1 à 5 et 8, **caractérisée en ce que** dans les produits de condensation d'éthanolamine-glycéroléther
R¹ représente un groupe alkyle comprenant 1 à 20, de préférence 3 à 20 et de manière particulièrement préférée 4 à 18 atomes de carbone, et
R² et R³ sont identiques et représentent un groupe de formule (IId) 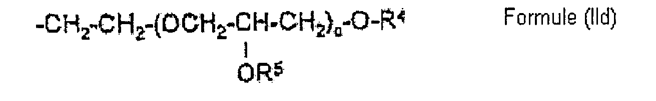 où
R⁵ représente hydrogène,
c représente un nombre de 1 à 25, de préférence de 1 à 20, de manière particulièrement préférée de 2 à 15, en particulier de préférence de 4 à 12, de manière extrêmement préférée de 6 à 10, et
R⁴ représente hydrogène.

15. Utilisation selon l'une ou plusieurs des revendications 1 à 5, 8 et 14, **caractérisée en ce que** dans les produits de condensation d'éthanolamine-glycéroléther
R¹ représente un groupe alkyle comprenant 1 à 10 atomes de carbone, de préférence un groupe n-butyle et
R² et R³ sont identiques et représentent un groupe de formule (IId) 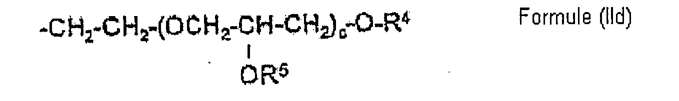 où
R⁵ représente hydrogène,
c représente un nombre de 1 à 25, de préférence de 1 à 20, de manière particulièrement préférée de 2 à 15, en particulier de préférence de 4 à 12, de manière extrêmement préférée de 6 à 10, et
R⁴ représente hydrogène.

16. Utilisation selon l'une ou plusieurs des revendications 1 à 15 dans des dispersions.

17. Produit de condensation d'éthanolamine-glycéroléther de formule (I) 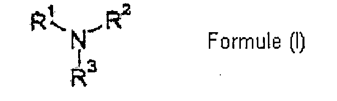 où
R¹, R² et R³ peuvent être identiques ou différents, à chaque fois indépendamment l'un de l'autre, et représentent hydrogène,
une chaîne alkyle linéaire ou ramifiée, saturée, comprenant 1 à 20, de préférence 3 à 20 et de manière particulièrement préférée 4 à 18 atomes de carbone,
un groupe alcényle linéaire ou ramifié, insaturé, comprenant 2 à 20, de préférence 3 à 20 et de manière particulièrement préférée 4 à 18 atomes de carbone,
-CH₂-CH₂-OH ou
un radical de formule (II) 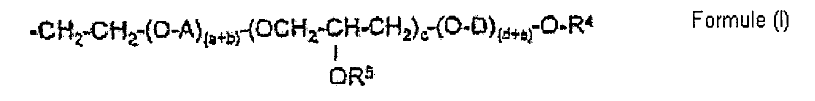 où
(O-A) représente un groupe d'alkylène-oxy choisi parmi le groupe d'éthylène-oxy (OE), le groupe de propylène-oxy (OP), le groupe de butylène-oxy et/ou le groupe de phényloxirane,
a représente un nombre de 0 à 50, de préférence de 0 à 20, de manière particulièrement préférée de 0 à 10,
b représente un nombre de 0 à 50, de préférence de 0 à 20, de manière particulièrement préférée de 0 à 10,
R⁵ représente hydrogène et/ou
(O-A)ₐH, où (O-A) représente un groupe d'alkylèneoxy choisi parmi le groupe d'éthylène-oxy (OE), le groupe de propylène-oxy (OP), le groupe de butylène-oxy et/ou le groupe de phényloxirane et a représente un nombre de 1 à 30 et/ou
-(CR⁶R⁷)ₐ-phényle, où R⁶ et R⁷, qui peuvent être identiques ou différents, représentent -H, un groupe OH, un groupe (C₁-C₁₀)-alkyle linéaire ou ramifié ou un groupe (C₂-C₃₀)-alcényle linéaire ou ramifié et a représente un nombre de 1 à 10, et/ou -(CR⁶R⁷)ₐ-naphtyle, où R⁶ et R⁷, qui peuvent être identiques ou différents, représentent -H, un groupe OH, un groupe (C₁-C₁₀)-alkyle linéaire ou ramifié ou un groupe (C₂-C₃₀)-alcényle linéaire ou ramifié et a représente un nombre de 1 à 10, et/ou un groupe de formule (III) 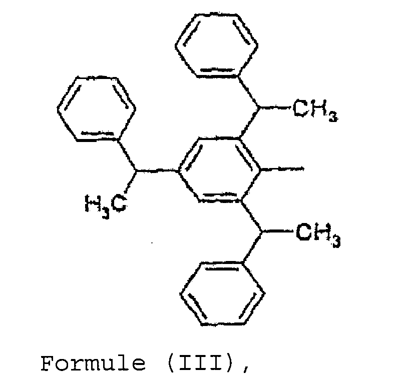 R⁸R⁹N-(CH₂)_{b}-, où R⁸ et R⁹, qui peuvent être identiques ou différents, représentent -H, un groupe (C₁-C₁₀)-alkyle linéaire ou ramifié ou un groupe (C₂-C₃₀)-alcényle linéaire ou ramifié et b représente un nombre de 1 à 22, et/ou HO-(CH₂)_{b}-, où b représente un nombre de 1 à 22, et/ou
-SO₃⁻X⁺, -PO₃²⁻X⁺X'⁺ ou -CH₂COO⁻X⁺, où X⁺ et X'⁺ représentent H⁺, Li⁺, Na⁺, K⁺, Ca²⁺/2, Mg²⁺/2 ou N(R⁸)₄⁺ et les radicaux R⁸ peuvent être identiques ou différents et représentent -H ou (C₁-C₁₀)-alkyle, de préférence (C₁-C₄)-alkyle, et/ou un groupe de formule (IV) 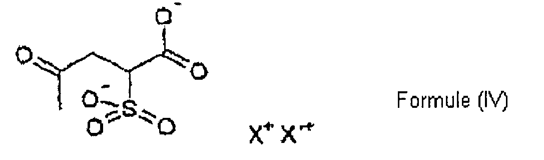 où X⁺ et X'⁺ représentent H⁺, Li⁺, Na⁺, K⁺, Ca²⁺/2, Mg²⁺/2 ou N(R⁸)₄⁺ et les radicaux R⁸ peuvent être identiques ou différents et représentent -H ou (C₁-C₁₀)-alkyle, de préférence (C₁-C₄)-alkyle, et/ou
-C(R¹¹)₂-COO⁻X⁺, -CO-R¹²-COO⁻X⁺ ou -C(R¹¹)₂C(R¹¹)₂C(R¹¹)₂-N(R¹³)₂, où les radicaux R¹¹ peuvent être identiques ou différents et représentent -H et/ou -CH₃, R¹² représente (C₁-C₁₀)-alkylène ou (C₂-C₃₀)-alcénylène, les radicaux R¹³ peuvent être identiques ou différents et représentent (C₁-C₁₀)-alkyle ou (C₂-C₃₀)-alcényle, X⁺ représente H⁺, Li⁺, Na⁺, K⁺, Ca²⁺/2, Mg²⁺/2 ou N(R⁸)₄⁺ et les radicaux R⁸ peuvent être identiques ou différents et représentent -H ou (C₁-C₁₀)-alkyle, de préférence (C₁-C₄)-alkyle, et/ou
-C(R¹⁴)₂C(R¹⁴)₂C(R¹⁴)₂-N((GO)_{z}H)₂, où les radicaux R¹⁴ peuvent être identiques ou différents et représentent -H et/ou -CH₃, G représente -C₂H₄-, -C₃H₆- ou -C₄H₈- et z représente un nombre de 1 à 22, et/ou
un groupe de formule (V) 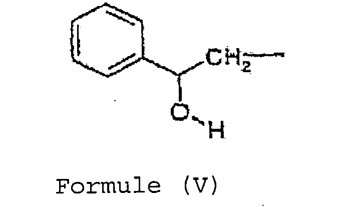
c représente un nombre de 1 à 25, de préférence un nombre de 1 à 20,
(O-D) représente un groupe d'alkylène-oxy choisi parmi le groupe d'éthylène-oxy (OE), le groupe de propylène-oxy (OP), le groupe de butylène-oxy et/ou le groupe de phényloxirane,
d représente un nombre de 0 à 50, de préférence de 0 à 20, de manière particulièrement préférée de 0 à 10,
e représente un nombre de 0 à 50, de préférence de 0 à 20, de manière particulièrement préférée de 0 à 10, et
R⁴ peut présenter la signification de R⁵, à condition qu'au moins deux des radicaux R¹, R² et R³ représentent des radicaux de formule (II) et qu'au moins un autre des radicaux R¹, R² et R³ soit choisi parmi -CH₂-CH₂-OH et les radicaux de formule (II).

18. Produit de condensation d'éthanolamine-glycéroléther selon la revendication 17, **caractérisé en ce que** deux des radicaux R¹, R² et R³ sont des radicaux de formule (II).

19. Produit de condensation d'éthanolamine-glycéroléther selon la revendication 18, **caractérisé en ce que** le troisième des radicaux R¹, R² et R³ représente une chaîne alkyle linéaire ou ramifiée, saturée, comprenant 1 à 20, de préférence 3 à 20 et de manière particulièrement préférée 4 à 18 atomes de carbone ou un groupe alcényle linéaire ou ramifié, insaturé comprenant 2 à 20, de préférence 3 à 20 et de manière particulièrement préférée 4 à 18 atomes de carbone.

20. Produit de condensation d'éthanolamine-glycéroléther selon la revendication 19, **caractérisé en ce que** le troisième des radicaux R¹, R² et R³ représente une chaîne alkyle linéaire ou ramifiée, saturée, comprenant 1 à 20, de préférence 3 à 20 et de manière particulièrement préférée 4 à 18 atomes de carbone.

21. Produit de condensation d'éthanolamine-glycéroléther selon la revendication 17, **caractérisé en ce que** les trois radicaux R¹, R² et R³ sont choisis parmi -CH₂-H₂-OH et les radicaux de formule (II).

22. Produit de condensation d'éthanolamine-glycéroléther selon la revendication 21, **caractérisé en ce que** les trois radicaux R¹, R² et R³ sont des radicaux de formule (II).

23. Produit de condensation d'éthanolamine-glycéroléther selon l'une ou plusieurs des revendications 17 à 22, **caractérisé en ce que** (O-A) et (O-D) représentent à chaque fois un groupe d'alkylèneoxy uniforme, (a+b) représente un nombre de 0 à 20 et (d+e) représente un nombre de 0 à 20.

24. Produit de condensation d'éthanolamine-glycéroléther selon l'une ou plusieurs des revendications 17 à 22, **caractérisé en ce que** (O-A)_{(a+b)} et (O-D)_{(d+e)} représentent des groupes d'alkylène-oxy mixtes de formule -(O-A¹)ₐ-(O-A²)_{b}- ou -(O-D¹)_{d}-(O-D²)ₑ-, qui peuvent être disposés de manière aléatoire ou par blocs et dans lesquels a et b signifient à chaque fois un nombre de 1 à 30 et d et e signifient à chaque fois un nombre de 1 à 20, (O-A¹) et (O-D¹) représentent une unité d'éthylène-oxy et (O-A²) et (O-D²) représentent une unité de propylène-oxy, une unité de butylène-oxy ou une unité de phényloxirane, ou des mélanges de ceux-ci.

25. Produit de condensation d'éthanolamine-glycéroléther selon l'une ou plusieurs des revendications 17 et 21 à 23, **caractérisé en ce que** les radicaux R¹, R² et R³ sont identiques et représentent un groupe de formule (IIa), 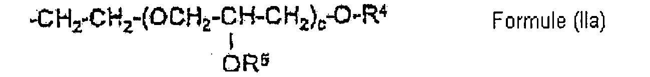 où
R⁵ représente hydrogène,
c représente un nombre de 1 à 25, de préférence de 1 à 20, de manière particulièrement préférée de 2 à 15, en particulier de préférence de 4 à 12, de manière extrêmement préférée de 6 à 10, et
R⁴ représente hydrogène.

26. Produit de condensation d'éthanolamine-glycéroléther selon l'une ou plusieurs des revendications 17 et 21 à 23, **caractérisé en ce que** les radicaux R¹, R² et R³ sont identiques et représentent un groupe de formule (IIa), 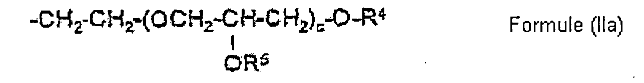 où
R⁵ représente hydrogène,
c représente un nombre de 1 à 25, de préférence de 1 à 20, de manière particulièrement préférée de 2 à 15, en particulier de préférence de 4 à 12, de manière extrêmement préférée de 6 à 10, et
R⁴ représente un groupe de formule (III)

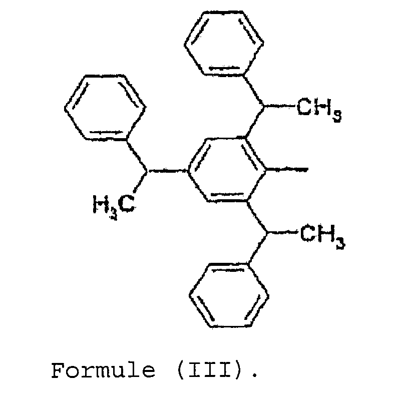

27. Produit de condensation d'éthanolamine-glycéroléther selon l'une ou plusieurs des revendications 17, 21 et 22, **caractérisé en ce que** les radicaux R¹, R² et R³ sont identiques et représentent un groupe de formule (IIb), 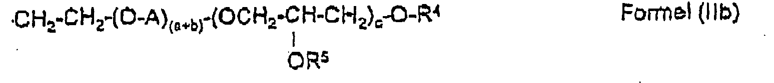 où
(O-A)_{(a+b)} représente un groupe d'alkylène-oxy uniforme et (a+b) représente un nombre de 1 à 50 ou un groupe d'alkylène-oxy mixte de formule -(O-A¹)ₐ-(O-A²)_{b}-, qui peut être disposé de manière aléatoire ou par blocs et où (a+b) représente un nombre de 1 à 50, (O-A¹) représente une unité d'éthylène-oxy et (O-A²) représente une unité de propylène-oxy, une unité de butylène-oxy, une unité de phényloxirane, ou les mélanges de ceux-ci,
R⁵ représente hydrogène,
c représente un nombre de 1 à 25, de préférence de 1 à 20, de manière particulièrement préférée de 2 à 15, en particulier de préférence de 4 à 12, de manière extrêmement préférée de 6 à 10, et
R⁴ représente hydrogène.

28. Produit de condensation d'éthanolamine-glycéroléther selon l'une ou plusieurs des revendications 17, 21, 22 et 27, **caractérisé en ce que** les radicaux R¹, R² et R³ sont identiques et représentent un groupe de formule (IIc), 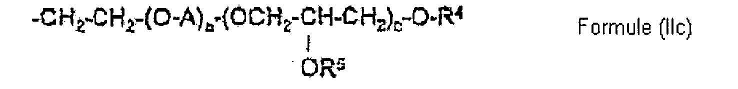 où
(O-A) représente un groupe d'alkylène-oxy, de préférence un groupe de propylène-oxy,
a représente un nombre de 1 à 50,
R⁵ représente hydrogène,
c représente un nombre de 1 à 25, de préférence de 1 à 20, de manière particulièrement préférée de 2 à 15, en particulier de préférence de 4 à 12, de manière extrêmement préférée de 6 à 10, et
R⁴ représente hydrogène.

29. Produit de condensation d'éthanolamine-glycéroléther selon l'une ou plusieurs des revendications 17 à 20 et 23, **caractérisé en ce que**
R¹ représente un groupe alkyle comprenant 1 à 20, de préférence 3 à 20 et de manière particulièrement préférée 4 à 18 atomes de carbone, et
R² et R³ sont identiques et représentent un groupe de formule (IId) 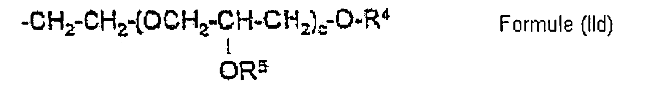 où
R⁵ représente hydrogène,
c représente un nombre de 1 à 25, de préférence de 1 à 20, de manière particulièrement préférée de 2 à 15, en particulier de préférence de 4 à 12, de manière extrêmement préférée de 6 à 10, et
R⁴ représente hydrogène.

30. Produit de condensation d'éthanolamine-glycéroléther selon l'une ou plusieurs des revendications 17 à 20, 23 et 29, **caractérisé en ce que** R¹ représente un groupe alkyle comprenant 1 à 10 atomes de carbone, de préférence un groupe n-butyle et
R² et R³ sont identiques et représentent un groupe de formule (IId) 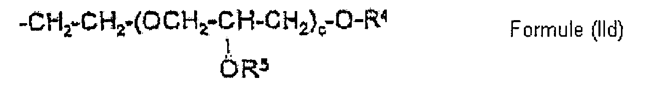 où
R⁵ représente hydrogène,
c représente un nombre de 1 à 25, de préférence de 1 à 20, de manière particulièrement préférée de 2 à 15, en particulier de préférence de 4 à 12, de manière extrêmement préférée de 6 à 10, et
R⁴ représente hydrogène.

31. Dispersion contenant un ou plusieurs produits de condensation d'éthanolamine-glycéroléther selon l'une ou plusieurs des revendications 17 à 30.
